# EUROPÄISCHE PATENTSCHRIFT

(11) **EP 1 194 403 B1**
(45) Veröffentlichungstag und Bekanntmachung des Hinweises auf die Patenterteilung: **03.09.2003**
(21) Anmeldenummer: 00940359.3
(22) Anmeldetag: 10.06.2000
(51) Int. Cl.: C07C 311/16, C07D 295/22, C07C 311/20, C07C 311/29, C07C 311/21, C07D 217/02, C07C 311/39, A61K 31/18, A61K 31/445

(54) **INDANYLSUBSTITUIERTE BENZOLCARBONAMIDE, VERFAHREN ZU IHRER HERSTELLUNG, IHRE VERWENDUNG ALS MEDIKAMENT SOWIE SIE ENTHALTENDE PHARMAZEUTISCHE ZUBEREITUNGEN**
INDANYL-SUBSTITUTED BENZOLE CARBONAMIDE, METHOD FOR THE PRODUCTION OF THE SAME, USE THEREOF AS A MEDICAMENT AND PHARMACEUTICAL PREPARATIONS CONTAINING THE SAME
BENZOLCARBONAMIDES SUBSTITUES PAR INDANYLE, LEUR PROCEDE DE PRODUCTION, LEUR UTILISATION COMME MEDICAMENT ET COMPOSITIONS PHARMACEUTIQUES LES CONTENANT

(30) Priorität: 25.06.1999 DE 19929076
(43) Veröffentlichungstag der Anmeldung: 10.04.2002
(73) Patentinhaber: Aventis Pharma Deutschland GmbH, 65929 Frankfurt am Main (DE)
(72) Erfinder: BRENDEL, Joachim, D-61118 Bad Vilbel (DE); GERLACH, Uwe, D-65795 Hattersheim (DE); STILZ, Hans, Ulrich, D-65929 Frankfurt (DE); LANG, Hans-Jochen, D-65719 Hofheim (DE)
(86) Internationale Anmeldenummer: EP0005370
(87) Internationale Veröffentlichungsnummer: WO01000573

(56) Entgegenhaltungen:
- EP-A- 0 258 096
- EP-A- 0 915 087
- WO-A-95/18617

## Beschreibung

Die Erfindung betrifft Verbindungen der Formel I. 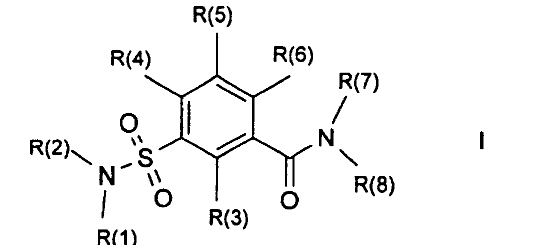 in der R(8) entweder für einen 1-Indanylrest der Formel II oder für einen 2-Indanylrest der Formel III steht, 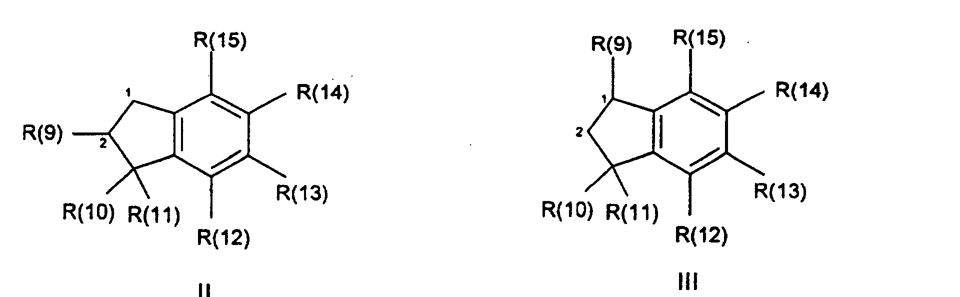 und worin R(1), R(2), R(3), R(4), R(5), R(6), R(7), R(9), R(10), R(11), R(12), R(13), R(14) und R(15) die im folgenden angegebenen Bedeutungen haben, ihre Herstellung und ihre Verwendung, insbesondere in Arzneimitteln.

Die erfindungsgemäßen Verbindungen wirken auf den sogenannten Kv1.5-Kalium-Kanal und inhibieren einen als "ultra-rapidly activating delayed rectifier" bezeichneten Kaliumstrom im humanen Herzvorhof. Die Verbindungen sind deshalb ganz besonders geeignet als neuartige antiarrhythmische Wirkstoffe, insbesondere zur Behandlung und Prophylaxe von Vorhof-Arrhythmien, z.B. Vorhofflimmern (atriale Fibrillation, AF) oder Vorhofflattern (atriales Flattern).

Vorhofflimmern (AF) und Vorhofflattern sind die häufigsten anhaltenden Herzarrhythmien. Das Auftreten erhöht sich mit zunehmenden Alter und führt häufig zu fatalen Folgeerscheinungen, wie zum Beispiel Gehirnschlag. AF betrifft ca. 1 Millionen Amerikaner jährlich und führt zu mehr als 80.000 Schlaganfällen jedes Jahr in den USA. Die zur Zeit gebräuchlichen Antiarrhythmika der Klasse I und III reduzieren die Wiederauftrittsrate von AF, finden aber wegen ihrer potentiellen proarrhythmischen Nebenwirkungen nur eingeschränkte Anwendung. Deshalb besteht eine hohe medizinische Notwendigkeit für die Entwicklung besserer Medikamente zur Behandlung atrialer Arrhythmien (S. Nattel, Am. Heart J. 130, 1995, 1094 - 1106; "Newer developments in the management of atrial fibrillation").

Es wurde gezeigt, daß den meisten supraventrikulären Arrhythmien sogenannte "Reentry" Erregungswellen unterliegen. Solche Reentries treten dann auf, wenn das Herzgewebe eine langsame Leitfähigkeit und gleichzeitig sehr kurze Refraktärperioden besitzt. Das Erhöhen der myokardialen Refraktärzeit durch Verlängerung des Aktionspotentials ist ein anerkannter Mechanismus, um Arrhythmien zu beenden bzw. deren Entstehen zu verhindern (T.J. Colatsky et al, Drug Dev. Res. 19, 1990, 129 - 140; "Potassium channels as targets for antiarrhythmic drug action"). Die Länge des Aktionspotentials wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" I_{K} zugeschrieben, der aus 3 verschiedenen Komponenten besteht: IKᵣ, IKₛ und IKᵤᵣ.

Die meisten bekannten Klasse III- Antiarrhythmika (z.B. Dofetilide, E4031 und d-Sotalol) blockieren überwiegend oder ausschließlich den schnell aktivierenden Kaliumkanal IKᵣ, der sich sowohl in Zellen des menschlichen Ventrikel als auch im Vorhof nachweisen läßt. Es hat sich jedoch gezeigt, daß diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D. M. Roden, Am. J. Cardiol. 72, 1993, 44B-49B; "Current status of class III antiarrhythmic drug therapy"). Neben diesem hohen, zum Teil tödlichen Risiko bei niedriger Frequenz, wurde für die I_{Kr}-Blocker ein Nachlassen der Wirksamkeit unter den Bedingungen von Tachykardie, in der die Wirkung gerade benötigt wird, festgestellt ("negative use-dependence").

Während einige dieser Nachteile durch Blocker der langsam aktivierenden Komponente (IKₛ) möglicherweise überwunden werden können, wurde deren Wirksamkeit bisher nicht bewiesen, da keine klinischen Untersuchungen mit IKₛ-Kanalblockern bekannt sind.

Die "besonders schnell" aktivierende und sehr langsam inaktivierende Komponente des delayed Rectifier IKᵤᵣ (=ultra-rapidly activating delayed rectifier), die dem Kv1.5-Kanal entspricht, spielt eine besonders große Rolle für die Repolarisationsdauer im menschlichen Vorhof. Eine Inhibierung des IKᵤᵣ-Kaliumauswärtsstroms stellt somit im Vergleich zur Inhibierung von IKᵣ bzw. IKₛ eine besonders effektive Methode zur Verlängerung des atrialen Aktionspotentials und damit zur Beendigung bzw. Verhinderung von atrialen Arrhythmien dar.

Im Gegensatz zu IKᵣ und IKₛ, die auch im menschlichen Ventrikel vorkommen, spielt der IKᵤᵣ zwar eine bedeutende Rolle im menschlichen Vorhof, jedoch nicht im Ventrikel. Aus diesem Grunde ist bei Inhibierung des IKᵤᵣ-Stroms im Gegensatz zur Blockade von IKᵣ oder IKₛ das Risiko einer proarrhythmischen Wirkung auf den Ventrikel von vornherein ausgeschlossen. (Z. Wang et al, Circ. Res. 73, 1993, 1061 - 1076: "Sustained Depolarisation-Induced Outward Current in Human Atrial Myocytes"; G.-R. Li et al, Circ. Res. 78, 1996, 689 - 696: "Evidence for Two Components of Delayed Rectifier K⁺-Current in Human Ventricular Myocytes"; G. J. Amos et al, J. Physiol. 491, 1996, 31 - 50: "Differences between outward currents of human atrial and subepicardial ventricular myocytes").

Selektive Blocker des IKᵤᵣ bzw. Kv1.5-Kanals sind in der Literatur bisher nicht beschrieben. Für zahlreiche pharmazeutische Wirkstoffe (z.B. Tedisamil, Bupivacaine oder Sertindole) wurde zwar eine blockierende Wirkung auf den Kv1.5-Kanal beschrieben, doch stellt die Kv1.5-Blockade hier jeweils nur eine Nebenwirkung neben anderen Hauptwirkungen der Substanzen dar. In WO 98 04 521 werden Aminoindane als Kaliumkanalblocker beansprucht, die den Kv1.5-Kanal blockieren. Allerdings wird für diese Verbindungen auch eine äquipotente Wirkung auf den Kv1.3-Kanal beschrieben. Eine Blockade des Kv1.3-Kanals, der eine Rolle in menschlichen T-Lymphozyten spielt, führt zu einer immunsuppressiven Wirkung, die als Nebenwirkung eines chronisch zu verabreichenden Antiarrhythmikums nicht erwünscht ist. In den Anmeldungen WO 98 18 475 und WO 98 18 476 wird die Verwendung verschiedener Pyridazinone und Phosphinoxide als Antiarrhythmika beansprucht, die über eine Blockade des IKᵤᵣ wirken sollen. Allerdings wurden diese Verbindungen ursprünglich (WO 96 25 936) ebenfalls als Immunsuppressiva beschrieben, so daß Ihre medizinische Verwendbarkeit zur Behandlung von Vorhofarrhythmien zweifelhaft erscheint.

Es wurde nun gefunden, daß die erfindungsgemäßen Verbindungen potente Blocker des humanen Kv1.5-Kanals sind. Sie können deshalb verwendet werden als neuartige Antiarrhythmika mit besonders vorteilhaftem Sicherheitsprofil. Insbesondere eignen sich die Verbindungen zur Behandlung supraventrikulärer Arrhythmien, z.B. Vorhofflimmem oder Vorhofflattern.

Die erfindungsgemäßen Verbindungen der Formel I sind bisher nicht bekannt. Einige strukturell verwandte Indanderivate sind beschrieben in den Anmeldungen EP 258 096 und EP 374 054. Die dort beanspruchten Verbindungen unterscheiden sich jedoch von den erfindungsgemäßen Verbindungen in dieser Anmeldung dadurch, daß in den genannten Anmeldungen R(9) für einen basischen Aminsubstituenten steht. Außerdem sind dort lediglich unsubstituierte Sulfonamide (R1 und R2 = H) beschrieben, während hier gefunden wurde, daß gerade substituierte Sulfonamide besonders wirksame Blocker des Kv1.5-Kanals sind.

Die vorliegende Erfindung betrifft Verbindungen der Formel I, 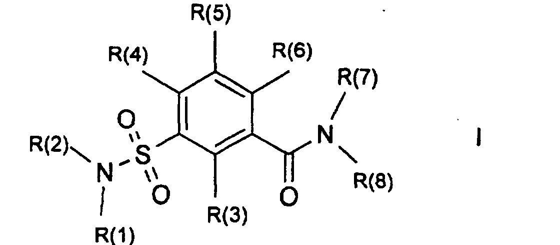 in der R(8) entweder für einen 1-Indanylrest der Formel II oder für einen 2-Indanylrest der Formel III steht, 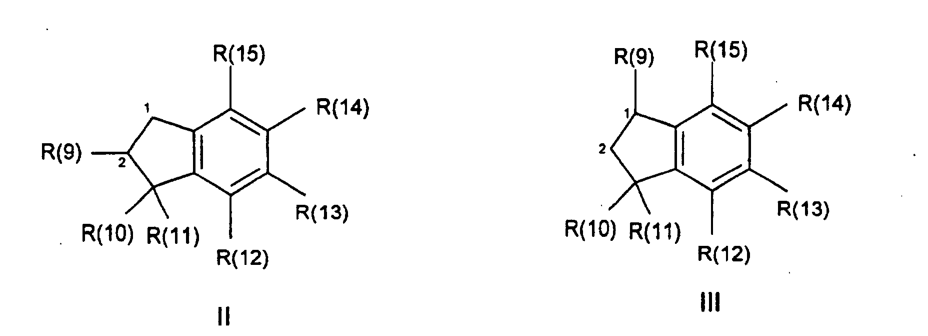 und worin bedeuten:
R(1) und R(2)
   unabhängig voneinander R(20)-CᵣH₂ᵣ,
   wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(21)- oder -CONR(21);
   R(21) Wasserstoff oder Alkyl mit 1, 2 oder 3 C-Atomen;
   R(20) H, CH₃, CH₂F, CHF₂, CF₃, C₂F₅, C₃F₇, Cycloalkyl mit 3, 4, 5, 6, 7 oder 8 C-Atomen, NR(22)R(23), -CONR(22)R(23), -OR(24), -COOR(24), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Hydroxymethyl, Hydroxyethyl, Methoxy, Dimethylamino, Sulfamoyl, Methylsulfonyl und Methylsulfonylamino;
   R(22) und R(23)
   unabhängig voneinander Wasserstoff oder Alkyl mit 1, 2, 3 oder 4 C-Atomen;
   oder R(22) und R(23)
   gemeinsam eine Kette von 4 oder 5 Methylengruppen, von denen eine CH₂-Gruppe durch -O-, -S-, -NH-, -N(Methyl)-oder -N(Benzyl)- ersetzt sein kann;
   R(24) Wasserstoff oder Alkyl mit 1, 2 oder 3 C-Atomen;
   r Null, 1, 2, 3, 4, 5, 6, 7 oder 8;
   oder
R(1) und R(2)
   gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
R(3), R(4), R(5) und R(6)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, NO₂, OR(25) oder NR(26)R(27);
   R(25) Wasserstoff, Alkyl mit 1, 2, 3 oder 4 C-Atomen, ein fluorierter Alkylrest der Formel -CₓH₂ₓCF_{y}H_{3-y} oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylaminos;
   x 0, 1, 2 oder 3;
   y 1, 2 oder 3;
   R(26) und R(27)
   unabhängig voneinander Wasserstoff oder Alkyl mit 1, 2, 3 oder 4 C-Atomen;
   oder
   R(26) und R(27)
   gemeinsam eine Kette von 4 oder 5 Methylengruppen, von denen eine CH₂-Gruppe durch -O-, -S-, -NH-, -N(Methyl)- oder -N(Benzyl)-ersetzt sein kann;
R(7) Wasserstoff oder Alkyl mit 1, 2, 3 oder 4 C-Atomen;
R(9) Wasserstoff, OR(28) oder OCOR(28);
   R(28) Wasserstoff oder Alkyl mit 1, 2, 3 oder 4 C-Atomen;
R(10) und R(11)
   unabhängig voneinander Wasserstoff oder Alkyl mit 1, 2, 3 oder 4 C-Atomen;
R(12), R(13), R(14) und R(15)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(29), Phenyl, Thienyl, Furyl oder ein N-haltiger Heterocyclus mit 1, 2, 3, 4, 5, 6, 7, 8 oder 9 C-Atomen,
   wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Y -O-, -CO-, -CO-O-, -O-CO-, -S-, -SO-, -SO₂-, -O-SO₂-, -SO₂NR(30)-, -CONR(30)- oder -NR(30)CO-, wobei die Verknüpfung mit dem Grundgerüst jeweils über das links stehende Atom erfolgt; R(30) Wasserstoff oder Alkyl mit 1, 2 oder 3 C-Atomen;
   s Null, 1, 2, 3, 4, 5 oder 6;
   R(29) Wasserstoff, Methyl, CF₃, C₂F₅, C₃F₇, Cycloalkyl mit 3, 4, 5, 6, 7 oder 8 C-Atomen, -OR(31), -COOR(31), -NR(32)R(33), -CONR(32)R(33), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(31) Wasserstoff oder Alkyl mit 1, 2 oder 3 C-Atomen;
   R(32) und R(33)
   unabhängig voneinander Wasserstoff oder Alkyl mit 1, 2, 3 oder 4 C-Atomen;
   oder
   R(32) und R(33)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
sowie ihre physiologisch verträglichen Salze.

Bevorzugt sind Verbindungen der Formel I, mit den oben angegebenen Bedeutungen, wobei jedoch mindestens einer der Reste R(1) oder R(2) eine andere Bedeutung als Wasserstoff hat.

Besonders bevorzugt sind Verbindungen der Formel I, in der R(8) entweder für einen 1-Indanylrest der Formel II oder für einen 2-Indanylrest der Formel III steht und worin bedeuten:
R(1) Wasserstoff;
R(2) R(20)-CᵣH₂ᵣ,
   wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(21)- oder -CONR(21);
   R(21) Wasserstoff oder Alkyl mit 1, 2 oder 3 C-Atomen;
   R(20) CH₃, CH₂F, CHF₂, CF₃, C₂F₅, C₃F₇, Cycloalkyl mit 3, 4, 5, 6, 7 oder 8 C-Atomen, NR(22)R(23), -CONR(22)R(23), -OR(24), -COOR(24), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Hydroxymethyl, Hydroxyethyl, Methoxy, Dimethylamino, Sulfamoyl, Methylsulfonyl und Methylsulfonylamino;
   R(22) und R(23)
   unabhängig voneinander Wasserstoff oder Alkyl mit 1, 2, 3 oder 4 C-Atomen;
   oder
   R(22) und R(23)
   gemeinsam eine Kette von 4 oder 5 Methylengruppen, von denen eine CH₂-Gruppe durch -O-, -S-, -NH-, -N(Methyl)-oder -N(Benzyl)- ersetzt sein kann;
   R(24) Wasserstoff oder Alkyl mit 1, 2 oder 3 C-Atomen;
   r Null, 1, 2, 3, 4, 5, 6, 7 oder 8;
R(3), R(4), R(5) und R(6)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, NO₂, OR(25) oder NR(26)R(27);
   R(25) Wasserstoff, Alkyl mit 1, 2, 3 oder 4 C-Atomen, ein fluorierter Alkylrest der Formel -CₓH₂ₓCF_{y}H_{3-y} oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   x 0, 1, 2 oder 3;
   y 1, 2 oder 3;
   R(26) und R(27)
   unabhängig voneinander Wasserstoff oder Alkyl mit 1, 2, 3 oder 4 C-Atomen;
   oder
   R(26) und R(27)
   gemeinsam eine Kette von 4 oder 5 Methylengruppen, von denen eine CH₂-Gruppe durch -O-, -S-, -NH-, -N(Methyl)- oder -N(Benzyl)-ersetzt sein kann;
R(7) Wasserstoff oder Alkyl mit 1, 2, 3 oder 4 C-Atomen;
R(9) Wasserstoff, OR(28) oder OCOR(28);
   R(28) Wasserstoff oder Alkyl mit1, 2, 3 oder 4 C-Atomen;
R(10) und R(11)
   unabhängig voneinander Wasserstoff oder Alkyl mit 1, 2, 3 oder 4 C-Atomen;
R(12), R(13), R(14) und R(15)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(29), Phenyl, Thienyl, Furyl oder ein N-haltiger Heterocyclus mit 1, 2, 3, 4, 5, 6, 7, 8 oder 9 C-Atomen,
   wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Y -O-, -CO-, -CO-O-, -O-CO-, -S-, -SO-, -SO₂-, -O-SO₂-, -SO₂NR(30)-, -CONR(30)- oder -NR(30)CO-, wobei die Verknüpfung mit dem Grundgerüst jeweils über das links stehende Atom erfolgt; R(30) Wasserstoff oder Alkyl mit 1, 2 oder 3 C-Atomen;
   s Null, 1, 2, 3, 4, 5 oder 6;
   R(29) Wasserstoff, Methyl, CF₃, C₂F₅, C₃F₇, Cycloalkyl mit 3, 4, 5, 6, 7 oder 8 C-Atomen, -OR(31), -COOR(31), -NR(32)R(33), -CONR(32)R(33), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsutfonylamino;
   R(31) Wasserstoff oder Alkyl mit 1, 2 oder 3 C-Atomen;
   R(32) und R(33)
   unabhängig voneinander Wasserstoff oder Alkyl mit 1, 2, 3 oder 4 C-Atomen;
   oder
   R(32) und R(33)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann; sowie ihre physiologisch verträglichen Salze.

Ganz besonders bevorzugt sind Verbindungen der Formel I, in der R(8) für einen 1-Indanylrest der Formel II steht, also Verbindungen der Formel I a 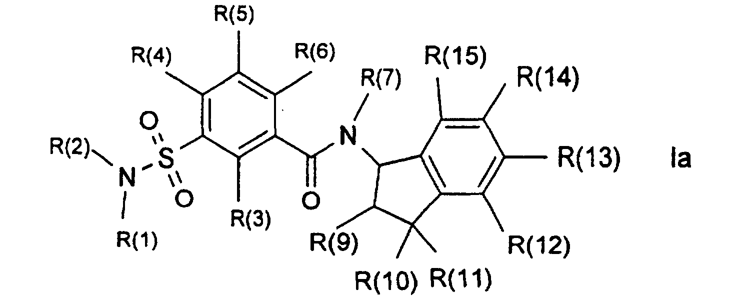 worin bedeuten:
R(1) Wasserstoff;
R(2) R(20)-CYᵣH₂ᵣ,
   wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(21)- oder -CONR(21);
   R(21) Wasserstoff oder Alkyl mit 1, 2 oder 3 C-Atomen;
   R(20) CH₃, CH₂F, CHF₂, CF₃, C₂F₅, C₃F₇, Cycloalkyl mit 3, 4, 5, 6, 7 oder 8 C-Atomen, NR(22)R(23), -CONR(22)R(23), -OR(24), -COOR(24), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Hydroxymethyl, Hydroxyethyl, Methoxy, Dimethylamino, Sulfamoyl, Methylsulfonyl und Methylsulfonylamino;
   R(22) und R(23)
   unabhängig voneinander Wasserstoff oder Alkyl mit 1, 2, 3 oder 4 C-Atomen;
   oder
   R(22) und R(23)
   gemeinsam eine Kette von 4 oder 5 Methylengruppen, von denen eine CH₂-Gruppe durch -O-, -S-, -NH-, -N(Methyl)-oder -N(Benzyl)- ersetzt sein kann;
   R(24) Wasserstoff oder Alkyl mit 1, 2 oder 3 C-Atomen;
   r Null, 1, 2, 3, 4, oder 5;
R(3), R(4), R(5) und R(6)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, NO₂ oder OR(25);
   R(25) Wasserstoff, Alkyl mit 1, 2, 3 oder 4 C-Atomen, ein fluorierter Alkylrest der Formel -CₓH₂ₓCF_{y}H_{3-y} oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   x 0, 1, 2 oder 3;
   y 1, 2 oder 3;
R(7) Wasserstoff oder Alkyl mit 1, 2, 3 oder 4 C-Atomen;
R(9) Wasserstoff, OR(28) oder OCOR(28);
   R(28) Wasserstoff oder Alkyl mit 1, 2, 3 oder 4 C-Atomen;
R(10) und R(11)
   unabhängig voneinander Wasserstoff oder Alkyl mit 1, 2, 3 oder 4 C-Atomen;
R(12), R(13), R(14) und R(15)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -NO₂, -Y-CₛH₂ₛ-R(29), Phenyl, Thienyl, Furyl oder ein N-haltiger Heterocyclus mit 1, 2, 3, 4, 5, 6, 7, 8 oder 9 C-Atomen,
   wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Y -O-, -CO-, -CO-O-, -O-CO-, -S-, -SO-, -SO₂-, -O-SO₂-, -SO₂NR(30)-, -CONR(30)- oder -NR(30)CO-, wobei die Verknüpfung mit dem Grundgerüst jeweils über das links stehende Atom erfolgt; R(30) Wasserstoff oder Alkyl mit 1, 2 oder 3 C-Atomen;
   s Null, 1, 2, 3, 4, 5 oder 6;
   R(29) Wasserstoff, Methyl, CF₃, C₂F₅, C₃F₇, Cycloalkyl mit 3, 4, 5, 6, 7 oder 8 C-Atomen, -OR(31), -COOR(31), -NR(32)R(33), -CONR(32)R(33), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(31) Wasserstoff oder Alkyl mit 1, 2 oder 3 C-Atomen;
   R(32) und R(33)
   unabhängig voneinander Wasserstoff oder Alkyl mit 1, 2, 3 oder 4 C-Atomen;
   oder
   R(32) und R(33)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
sowie ihre physiologisch verträglichen Salze.

Speziell bevorzugt sind Verbindungen der Formel I a, worin bedeuten:
R(1) Wasserstoff;
R(2) R(20)-CᵣH₂ᵣ;
   R(20) CH₃, CH₂F, CHF₂, CF₃, Cycloalkyl mit 3, 4, 5, 6, 7 oder 8 C-Atomen, -CONR(22)R(23), -OR(24), -COOR(24) oder Phenyl,
   das unsubstituiert ist oder substituiert mit 1 oder 2 Substituenten, ausgewählt aus der Gruppe bestehend aus F, Cl, Br, CF₃, NO₂, CN, OH, Methyl, Ethyl, Hydroxymethyl, Hydroxyethyl, Methoxy, Dimethylamino, Sulfamoyl, Methylsulfonyl und Methylsulfonylamino;
   R(22) und R(23)
   unabhängig voneinander Wasserstoff oder Alkyl mit 1, 2, 3 oder 4 C-Atomen;
   oder
   R(22) und R(23)
   gemeinsam eine Kette von 4 oder 5 Methylengruppen, von denen eine CH₂-Gruppe durch -O-, -S-, -NH-, -N(Methyl)-oder -N(Benzyl)- ersetzt sein kann;
   R(24) Wasserstoff oder Alkyl mit 1, 2, oder 3 C-Atomen;
   r Null, 1, 2, 3, 4, oder 5;
R(3), R(4), R(5) und R(6)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, NO₂ oder OR(25);
   R(25) Wasserstoff, Alkyl mit 1, 2, 3 oder 4 C-Atomen, ein fluorierter Alkylrest der Formel -CₓH₂ₓCF_{y}H_{3-y} oder Phenyl, das unsubstituiert ist oder substituiert mit 1 oder 2 Substituenten, ausgewählt aus der Gruppe bestehend aus F, Cl, Br, CF₃, NO₂, CN, OH, Methyl, Ethyl, Methoxy, Dimethylamino, Sulfamoyl, Methylsulfonyl und Methylsulfonylamino;
   x 0, 1, 2 oder 3;
   y 1, 2 oder 3;
R(7) Wasserstoff oder Alkyl mit 1, 2, 3 oder 4 C-Atomen;
R(9) Wasserstoff oder OR(28);
   R(28) Wasserstoff oder Alkyl mit 1, 2, 3 oder 4 C-Atomen;
R(10) und R(11)
   unabhängig voneinander Wasserstoff oder Alkyl mit 1, 2, 3 oder 4 C-Atomen;
R(12), R(13), R(14) und R(15)
   unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, -NO₂ oder -Y-CₛH₂ₛ-R(29);
   Y -O-, -CO-, -CO-O-, -O-CO-, -S-, -SO-, -SO₂-, -O-SO₂-, -SO₂NR(30)-, -CONR(30)- oder -NR(30)CO-, wobei die Verknüpfung mit dem Grundgerüst jeweils über das links stehende Atom erfolgt; R(30) Wasserstoff oder Alkyl mit 1, 2 oder 3 C-Atomen;
   s Null, 1, 2, 3, 4 oder 5;
   R(29) Wasserstoff, Methyl, CF₃, -OR(31), -COOR(31), -NR(32)R(33), -CONR(32)R(33) oder Phenyl,
   das unsubstituiert ist oder substituiert mit 1 oder 2 Substituenten, ausgewählt aus der Gruppe bestehend aus F, Cl, Br, CF₃, NO₂, CN, OH, Methyl, Ethyl, Methoxy, Dimethylamino, Sulfamoyl, Methylsulfonyl und Methylsulfonylamino;
   R(31) Wasserstoff oder Alkyl mit 1, 2 oder 3 C-Atomen;
   R(32) und R(33)
   unabhängig voneinander Wasserstoff oder Alkyl mit 1, 2, 3 oder 4 C-Atomen;
   oder
   R(32) und R(33)
   gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
sowie ihre physiologisch verträglichen Salze.

Alkylreste und Alkylenreste können geradkettig oder verzweigt sein. Dies gilt auch für die Alkylenreste der Formeln CᵣH₂ᵣ, CₛH₂ₛ und CₓH₂ₓ. Alkylreste und Alkylenreste können auch geradkettig oder verzweigt sein, wenn sie substituiert sind oder in anderen Resten enthalten sind, z. B. in einem Alkoxyrest oder in einem Alkylmercaptorest oder in einem fluorierten Alkylrest. Beispiele für Alkylreste sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,4-Butylen, 1,5-Pentylen, 2,2-Dimethyl-1,3-propylen, 1,6-Hexylen, usw. sind Beispiele für Alkylenreste.

Cycloalkylreste können ebenfalls verzweigt sein. Beispiele für Cycloalkylreste mit 3 bis 8 C-Atomen sind Cyclopropyl, Cyclobutyl, 1-Methylcyclopropyl, 2-Methylcyclopropyl, Cyclopentyl, 2-Methylcyclobutyl, 3-Methylcyclobutyl, Cyclopentyl, Cyclohexyl, 2-Methylcyclohexyl, 3-Methylcyclohexyl, 4-Methylcyclohexyl, Cycloheptyl, Cyclooctyl usw.

Als N-haltige Heterocyclen mit 1, 2, 3, 4, 5, 6, 7, 8 oder 9 C-Atomen gelten insbesondere die aromatischen Systeme 1-, 2- oder 3- Pyrrolyl, 1-, 2-, 4- oder 5-Imidazolyl, 1-, 3-, 4- oder 5-Pyrazolyl, 1,2,3-Triazol-1-, -4- oder -5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder-5-yl, 1,2,4-Oxadiazol-3-oder-5-yl, 1,3,4-Oxadiazol-2-yl oder -5-yl, 2-, 4- oder 5-Thiazolyl, 3-, 4- oder 5-Isothiazolyl, 1,3,4-Thiadiazol-2- oder -5-yl, 1,2,4-Thiadiazol-3- oder -5-yl, 1,2,3-Thiadiazol-4- oder -5-yl, 2-, 3- oder -4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl.

Besonders bevorzugt sind die N-haltigen Heterocyclen Pyrrolyl, Imidazolyl, Chinolyl, Pyrazolyl, Pyridyl, Pyrazinyl, Pyrimidinyl und Pyridazinyl.

Thienyl steht sowohl für 2- als auch 3-Thienyl.

Monosubstituierte Phenylreste können in der 2-, der 3- oder der 4-Position substituiert sein, disubstituierte in der 2,3-, 2,4-, 2,5-, 2,6-, 3,4- oder 3,5-Position. Entsprechendes gilt sinngemäß analog auch für die N-haltigen Heterocylen oder den Thiophenrest.

Bei Disubstitution eines Restes können die Substituenten gleich oder verschieden sein.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. gehören also in enantiomerenreiner Form, sowohl als links- als auch als rechtsdrehende Antipoden, und auch in Form von Mischungen der beiden Enantiomeren in unterschiedlichen Verhältnissen oder in Form von Racematen zu der Erfindung. Bei Vorliegen einer cis/trans-Isomerie gehören sowohl die cis-Form als auch die trans-Form und Gemische dieser Formen zur Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfaßt die vorliegende Erfindung auch alle tautomeren Formen der Verbindungen der Formel I.

Die Verbindungen der Formel I sind durch unterschiedliche chemische Verfahren herstellbar, die ebenfalls zu der vorliegenden Erfindung gehören. So erhält man beispielsweise eine Verbindung der Formel I, indem man
eine Carbonsäure der Formel IV, 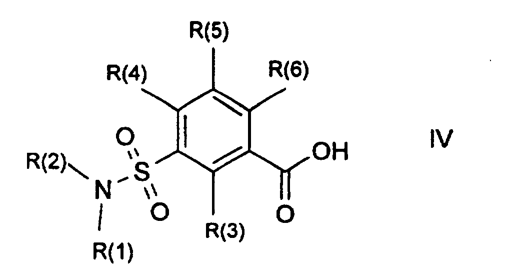 worin R(1), R(2), R(3), R(4), R(5) und R(6) die oben angegebenen Bedeutungen besitzen, in an sich bekannter Weise in einer Amidierungsreaktion mit einem Amin der Formel V a oder V b umsetzt, 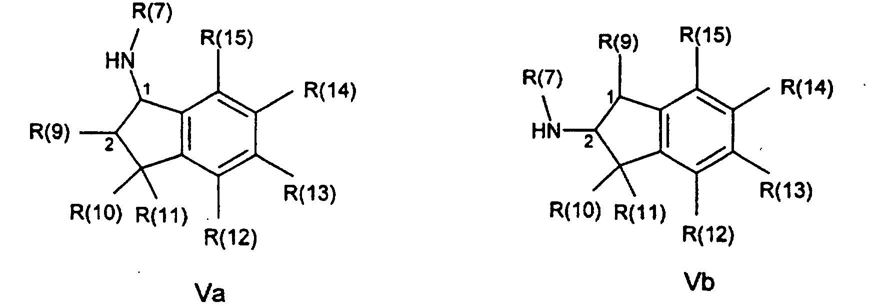 worin R(7), R(9), R(10), R(11), R(12), R(13), R(14) und R(15) die oben angegebenen Bedeutungen besitzen.

Zur Durchführung dieser Reaktionen sind in der Literatur zahlreiche Methoden beschrieben worden. Besonders vorteilhaft können sie durch Aktivierung der Carbonsäure, z.B. mit Dicyclohexylcarbodiimid (DCC), gegebenenfalls unter Zusatz von Hydroxybenzotriazol (HOBT) oder Dimethylaminopyridin (DMAP), oder mit O-[(Cyano(ethoxycarbonyl)methylen)amino]-1,1,3,3-tetramethyluroniumtetrafluoroborat (TOTU), durchgeführt werden. Es können aber auch zunächst nach bekannten Methoden reaktive Säurederivate synthetisiert werden, z.B. Säurechloride durch Umsetzung der Carbonsäuren der Formel IV mit anorganischen Säurehalogeniden, wie z.B. SOCI₂, oder Säureimidazolide durch Umsetzung mit Carbonyldiimidazol, die dann anschließend, gegebenenfalls unter Zusatz einer Hilfsbase, mit den Aminen der Formeln V a oder V b umgesetzt werden.

Die Amine der Formeln V a oder V b sind entweder literaturbekannt oder können in Analogie zu bekannten Methoden hergestellt werden, z.B. durch reduktive Aminierung der entsprechenden 1-lndanone oder durch Epoxidierung der entsprechenden 1H-Indene und anschließende Epoxidöffnung mit einem Amin der Formel R(7)-NH₂.

Die Carbonsäuren der Formel IV können z.B. erhalten werden aus den Chlorsulfonylverbindungen der Formel VI, 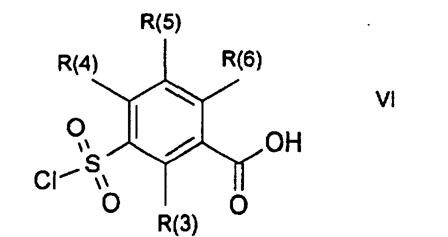 durch Umsetzung mit einem Amin der Formel R(1)R(2)NH in einem geeigneten inerten Lösungsmittel wie z. B. Diethylether, THF oder Aceton und ggf. in Gegenwart einer Hilfsbase wie z.B. Triethylamin.

Die Chlorsulfonylverbindungen der Formel VI sind entweder literaturbekannt oder können in Analogie zu bekannten Methoden hergestellt werden, z.B. durch Chlorsulfonierung entsprechend substituierter Benzoesäuren mit Chlorsulfonsäure.

Bei allen Verfahrensweisen kann es angebracht sein, bei bestimmten Reaktionsschritten funktionelle Gruppen im Molekül zeitweilig zu schützen. Solche Schutzgruppentechniken sind dem Fachmann geläufig. Die Auswahl einer Schutzgruppe für in Betracht kommende Gruppen und die Verfahren zu ihrer Einführung und Abspaltung sind in der Literatur beschrieben und können gegebenenfalls ohne Schwierigkeiten dem Einzelfall angepaßt werden.

Die erfindungsgemäßen Verbindungen der Formel I und ihre physiologisch verträglichen Salze können somit am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Verwendung zur Herstellung von Medikamenten dafür und von Medikamenten mit K⁺-Kanal-blockierender Wirkung. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen, pharmazeutisch einwandfreien Träger- und Hilfsstoffen enthalten. Die pharmazeutischen Zubereitungen enthalten normalerweise 0,1 bis 90 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnem, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Die Verbindungen der Formel I können zur Erzielung einer vorteilhaften therapeutischen Wirkung auch mit anderen Arzneiwirkstoffen kombiniert werden. So sind in der Behandlung von Herz-Kreislauferkrankungen vorteilhafte Kombinationen mit herz-kreislaufaktiven Stoffen möglich. Als derartige, für Herz-Kreislauferkrankungen vorteilhafte Kombinationspartner kommen beispielsweise andere Antiarrhythmika, so Klasse I-, Klasse II- oder Klasse III-Antiarrhythmika, in Frage, wie beispielsweise IKₛ- oder IKᵣ-Kanalblocker, z.B. Dofetilid, oder weiterhin blutdrucksenkende Stoffe wie ACE-Inhibitoren (beispielsweise Enalapril, Captopril, Ramipril), Angiotensin-Antagonisten, K⁺-Kanalaktivatoren, sowie alpha- und beta-Rezeptorenblocker, aber auch sympathomimetische und adrenerg wirkende Verbindungen, sowie Na⁺/H⁺-Austausch-Inhibitoren, Calciumkanalantagonisten, Phosphodiesterasehemmer und andere positiv inotrop wirkende Stoffe, wie z. B. Digitalisglykoside, oder Diuretika.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wäßrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I und ihre physiologisch verträglichen Salze können auch lyophilisiert werden und die erhaltenen Lyophilisate z. B. zur Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. Üblicherweise beträgt die tägliche Dosis einer Verbindung der Formel I bei Verabreichung an einem etwa 75 kg schweren Patienten 0.001 mg/kg Körpergewicht bis 100 mg/kg Körpergewicht, bevorzugt 0.01 mg/kg Körpergewicht bis 20 mg/kg Körpergewicht. Die Dosis kann in Form einer Einzeldosis verabreicht werden oder in mehrere, z. B. zwei, drei oder vier Einzeldosen aufgeteilt werden. Insbesondere bei der Behandlung akuter Fälle von Herzrhythmusstörungen, beispielsweise auf einer Intensivstation, kann auch eine parenterale Verabreichung durch Injektion oder Infusion, z. B. durch eine intravenöse Dauerinfusion, vorteilhaft sein.

### Experimenteller Teil

### Liste der Abkürzungen

- DMF: N,N-Dimethylformamid
- EE: Essigsäureethylester
- F.p.: Schmelzpunkt (Wenn nicht anders angegeben sind die Schmelzpunkte der ungereinigten Rohprodukte angegeben; die Schmelzpunkte der jeweiligen Reinsubstanzen können durchaus deutlich höher liegen)
- HOBT: 1-Hydroxy-1 H-benzotriazol
- i. Vak.: im Vakuum
- LM: Lösungsmittel
- RT: Raumtemperatur
- THF: Tetrahydrofuran
- TOTU: O-[(Cyano(ethoxycarbonyl)methylen)amino]-1,1,3,3-tetramethyluroniumtetrafluoroborat

### Allgemeine Vorschrift zur Synthese von 3-Chlorsulfonylbenzoesäuren (Formel VI)

0,2 mol einer substituierten Benzoesäure werden in 135 ml Chlorsulfonsäure eingetragen und die Reaktionsmischung wird 4 h auf 120°C erhitzt. Nach dem Abkühlen gießt man auf 800 g Eis, rührt 1 h nach und saugt das ausgefallene Produkt ab.

Auf diese Weise wurden u. a. folgende 3-Chlorsulfonylbenzoesäuren synthetisiert:

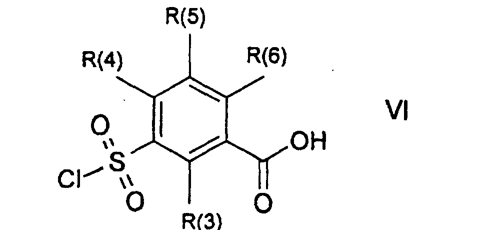

| R(3) | R(4) | R(5) | R(6) | Ausbeute | Fp. [°C] |
|---|---|---|---|---|---|
| H | Me | H | H | 93% | 174 |
| H | H | H | Cl | 91% | 148 |
| H | H | H | Me | 95% | 151 |
| H | F | H | H | 81% | 140 |
| H | Cl | H | H | 86% | 158 |
| H | Cl | H | Cl | 83% | 185 |
| H | F | H | Cl | 82% | 139 |
| H | Cl | NO₂ | Cl | 76% | 235 |
| H | H | NO₂ | Cl | 42% | 190 |
| H | Cl | Cl | H | 65% | 207 |
| H | OMe | H | H | 75% | |
| H | H | H | OMe | 80% | 145 |
| H | iPr | H | H | 83% | 192 |
| H | H | Me | Me | 82% | 170 |
| H | Me | H | Me | 88% | 190 |
| H | Me | Me | H | 81% | |

### Allgemeine Vorschrift zur Umsetzung von 3-Chlorsulfonylbenzoesäuren (Formel VI) mit Aminen zu Sulfonamiden der Formel IV (Variante A):

Zu einer Lösung von 30 mmol des entsprechenden Amins in 20 ml Diethylether (oder Methylenchlorid) werden 10 mmol der jeweiligen 3-Chlorsulfonylbenzoesäure zugegeben und die Reaktionsmischung wird über Nacht bei RT gerührt. Nach Zugabe von 20 ml Ether (bzw. Methylenchlorid) und 20 ml Wasser wird die organische Phase 2mal mit verdünnter Salzsäure und anschließend 2mal mit gesättigter Natriumbicarbonatlösung extrahiert. Nach Ansäuern der Bicarbonatextrakte fällt das Produkt der Formel IV aus und wird entweder durch Absaugen oder durch Extraktion mit EE isoliert.

### Auf diese Weise wurden u. a. folgende Sulfonamide der Formel IV synthetisiert:

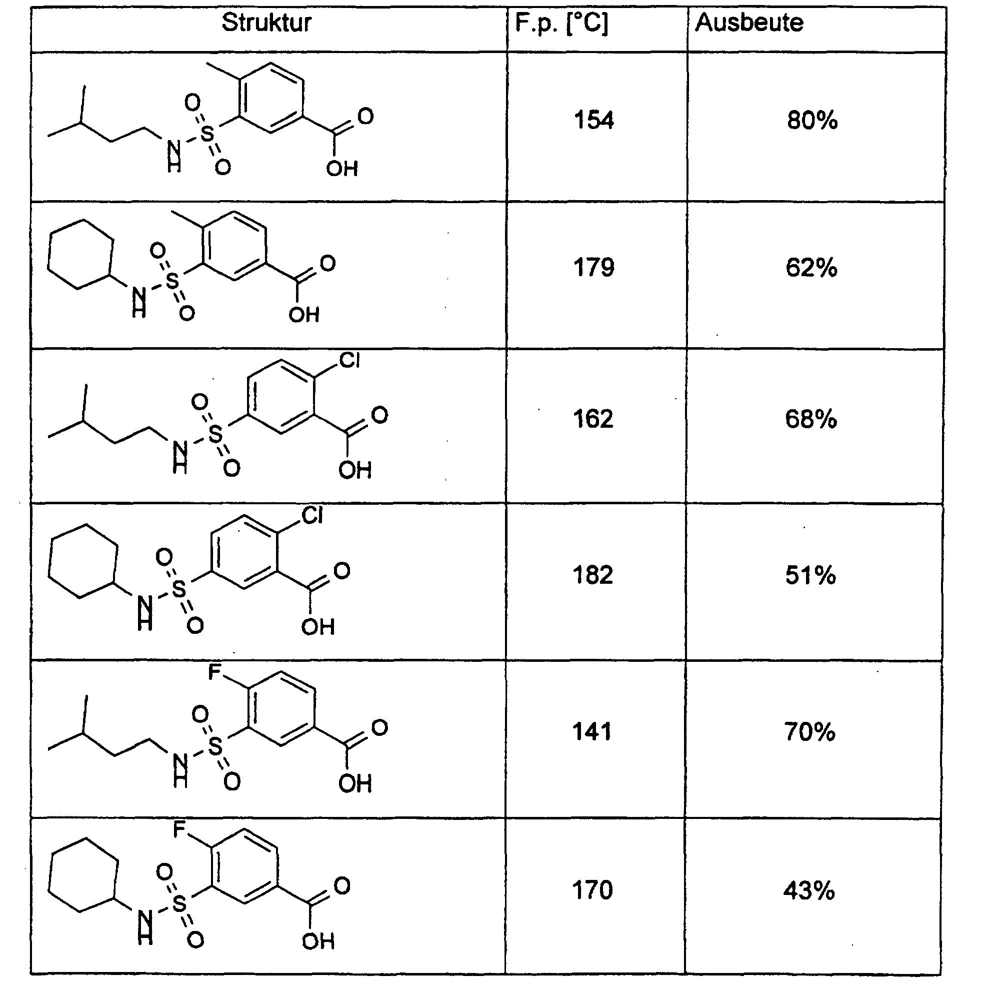

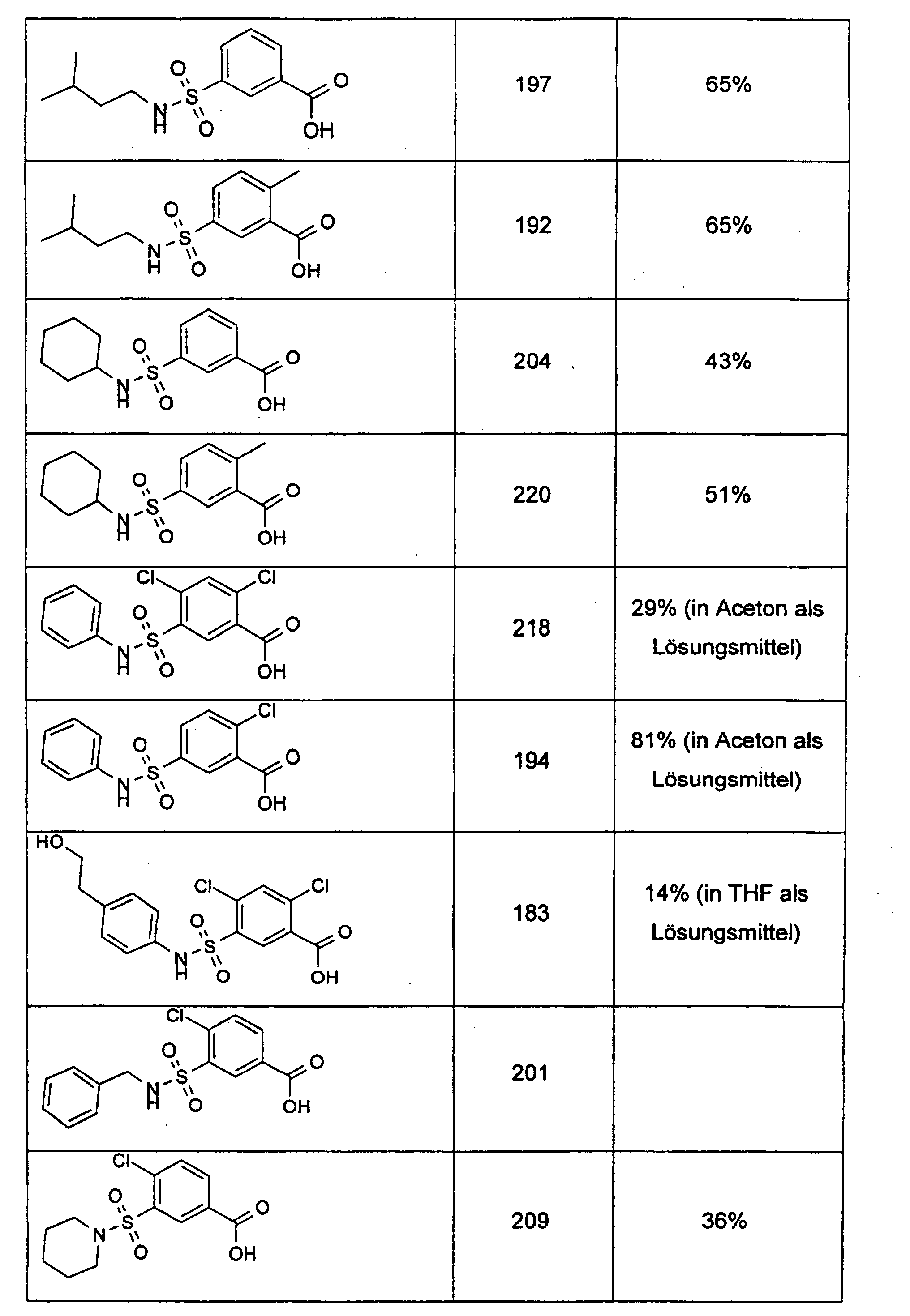

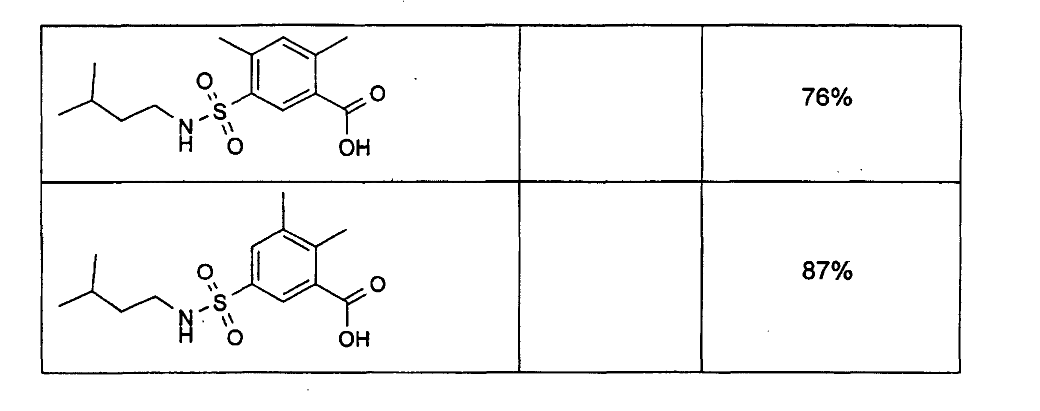

### Allgemeine Vorschrift zur Synthese von erfindungsgemäßen Verbindungen der Formel I aus 3-Chlorsulfonylbenzoesäuren der Formel VI (Variante B):

3 mmol eines Amins der Formel HNR(1)R(2) werden in 20 - 50 ml Diethylether gelöst und mit 1 mmol der 3-Chlorsulfonylbenzoesäure (Formel VI) gelöst in Ether versetzt. Man rührt 30 min bei RT, stellt mit 2M Salzsäure auf pH 1 und trennt die Phasen. Die org. Phase wird getrocknet, filtriert und i. Vak. eingedampft. Der Rückstand wird mit 5 ml Thionylchlorid 40 min unter Rückfluß gekocht. Anschließend entfernt man das überschüssige Thionylchlorid i. Vak. und koevaporiert mehrmals mit Toluol. Das so erhaltene Produkt wird in Ether oder Dichlormethan aufgenommen und zu einer Lösung von 1 mmol Aminoindan (Formel II oder III) und **1** mmol Hünigbase in Ether gegeben und 20 min bei RT gerührt. Anschließend stellt man mit 2M HCI auf pH 1 und trennt die Phasen. Die organische Phase wird getrocknet, filtriert und i. Vak. eingedampft. Das Rohprodukt wird durch Flash-Chromatographie gereinigt.

### Allgemeine Vorschrift zur Synthese von erfindungsgemäßen Verbindungen der Formel I aus Sulfonamiden der Formel IV (Variante C):

1 mmol einer Verbindung der Formel IV wird mit 5 ml Thionylchlorid 40 min unter Rückfluß gekocht. Anschließend entfernt man das überschüssige Thionylchlorid i. Vak. und koevaporiert mehrmals mit Toluol. Das so erhaltene Produkt wird in Ether oder Dichlormethan aufgenommen und zu einer Lösung von 10 mmol Aminoindan in Ether gegeben und bis zur vollständigen Umsetzung bei RT gerührt. Nach Einengen der Reaktionsmischung wird der Rückstand über präparative HPLC gereinigt.

### Allgemeine Vorschrift zur Synthese von erfindungsgemäßen Verbindungen der Formel I aus Sulfonamiden der Formel IV (Variante D):

Zu einer Lösung oder Suspension von 1 mmol einer Verbindung der Formel IV in THF werden 1,15 bis 1,40 mmol Carbonyldiimidazol hinzugefügt und die Reaktionsmischung wird 3 h bei RT gerührt. Nach Zugabe von 1,1 bis 1,5 mmol eines 1- oder 2-Aminoindans der Formel V a bzw. V b wird über Nacht bei RT gerührt und anschließend die Reaktionsmischung im Vakuum eingeengt. Der Rückstand wird in EE aufgenommen, und mit verd. Salzsäure und Natriumbicarbonatlösung gewaschen. Nach Einengen der organischen Phase im Vakuum, wird der Rückstand in Isopropanol gelöst und das Produkt durch Zugabe von Wasser ausgefällt. Nach Absaugen und Trocknen werden die Verbindungen der Formel I in einer Reinheit von >90% erhalten (zum Teil verunreinigt mit bis zu 10% des entsprechenden Bisindanylhamstoffs).

Allgemeine Vorschrift zur Synthese von erfindungsgemäßen Verbindungen der Formel I aus Sulfonamiden der Formel IV (Variante E):

1 mmol einer Verbindung der Formel IV wird in Gegenwart von 1 mmol TOTU, 1 mmol Hünigbase mit 1 mmol eines 1- oder 2-Aminoindans der Formel Va bzw. Vb in DMF umgesetzt. Nach 2 h Rühren bei RT wird das Lösungsmittel im Vakuum entfernt und das Rohprodukt durch Chromatographie am RP18-Kieselgel gereinigt.

### Allgemeine Vorschrift zur Synthese von erfindungsgemäßen Verbindungen der Formel I aus Sulfonamiden der Formel IV (Variante F):

Zu einer Lösung von 1,4 mmol einer Verbindung der Formel IV, 0,21 g (1,55 mmol) HOBT und 0.19 g (1,55 mmol) Diisopropylcarbodiimid in 15 ml THF werden bei 0°C 1,55 mmol eines 1- oder 2-Aminoindans der Formel V a bzw. V b zugegeben und die Reaktionsmischung wird über Nacht bei RT gerührt. Nach Abfiltrieren des ausgefallenen Niederschlages wird das Filtrat im Vakuum eingeengt und in EE aufgenommen und mit Natriumbicarbonatlösung extrahiert.

Nach den allgemeinen Vorschriften (Varianten B, C, D, E oder F) wurden u. a. die folgenden Verbindungen der Formel I synthetisiert, die in nachfolgender Tabelle aufgeführt sind.
Verbindungen, bei denen kein Schmelzpunkt angegeben ist, wurden als Öle oder amorph glasartige Produkte isoliert. Verbindungen bei denen kein Hinweis auf die verwendete Synthesevariante enthalten ist, wurden in Analogie zu den beschriebenen Methoden erhalten, jedoch unter Anwendung kleiner Variationen, wie z. B. andere Lösungsmittel, anderes Kupplungsreagenz, etc. Eine zwingende Notwendigkeit für die Verwendung einer bestimmten Methode ist in keinem Fall gegeben, sondern in der Regel können alle Varianten verwendet werden.

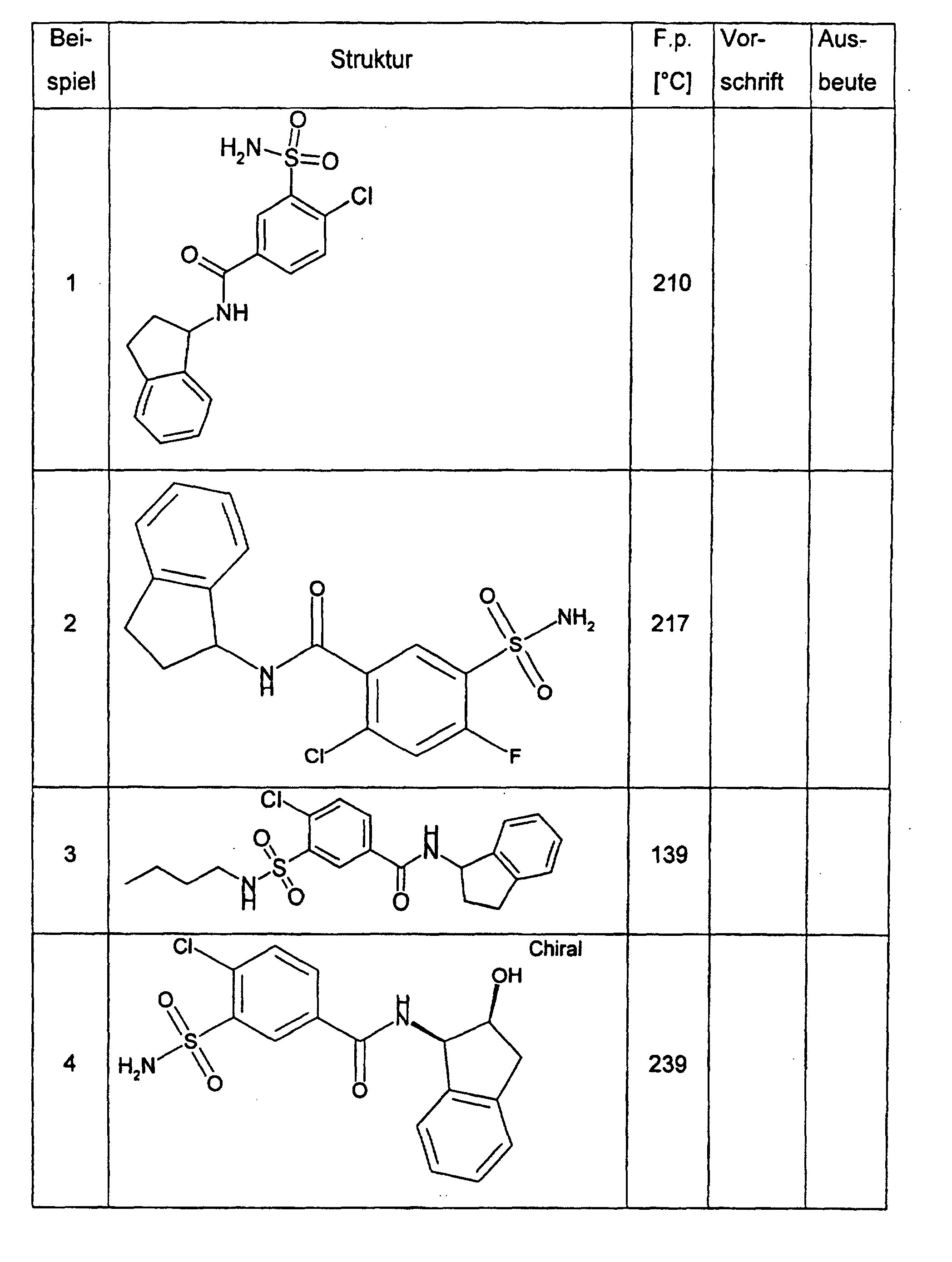

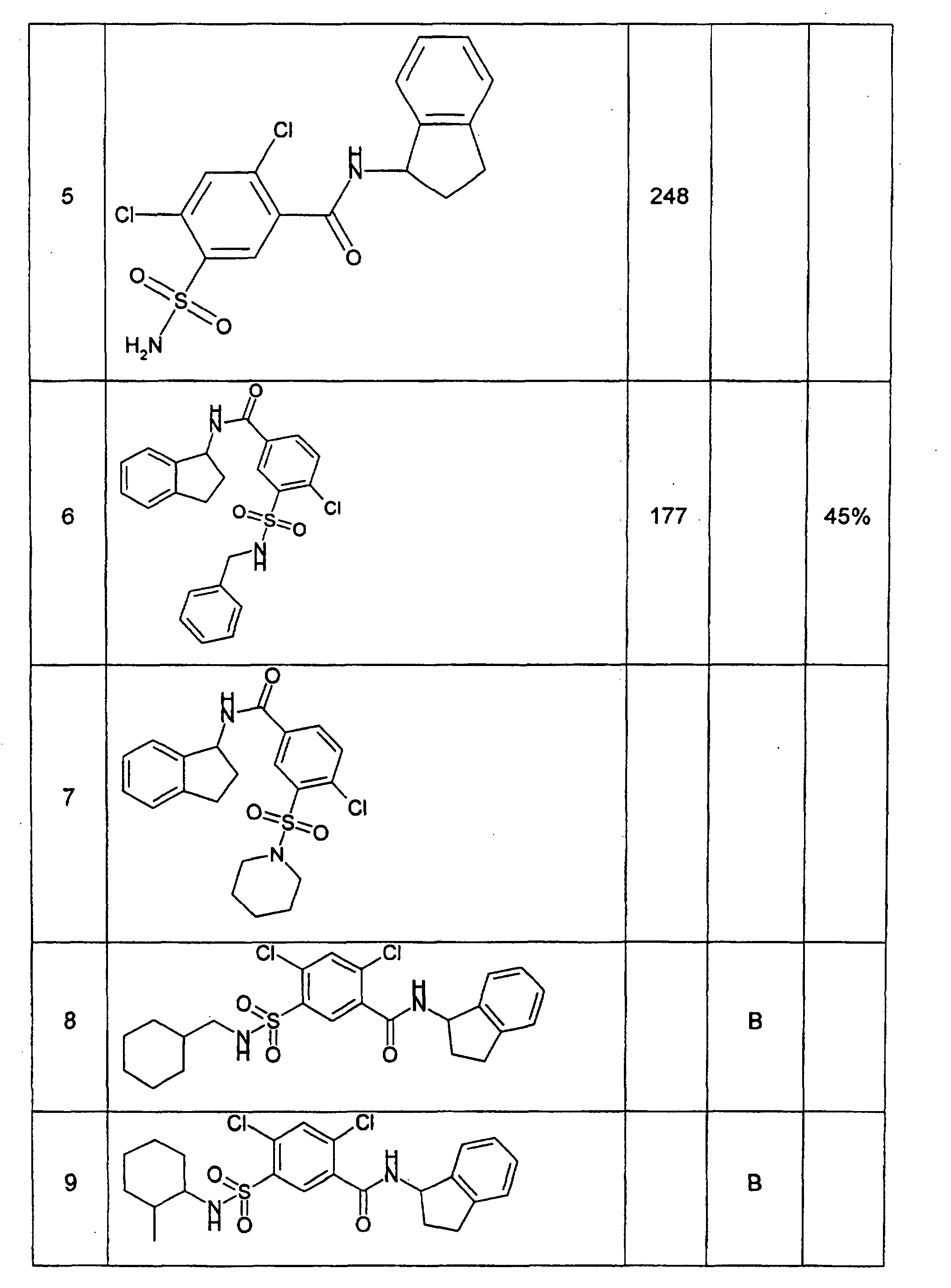

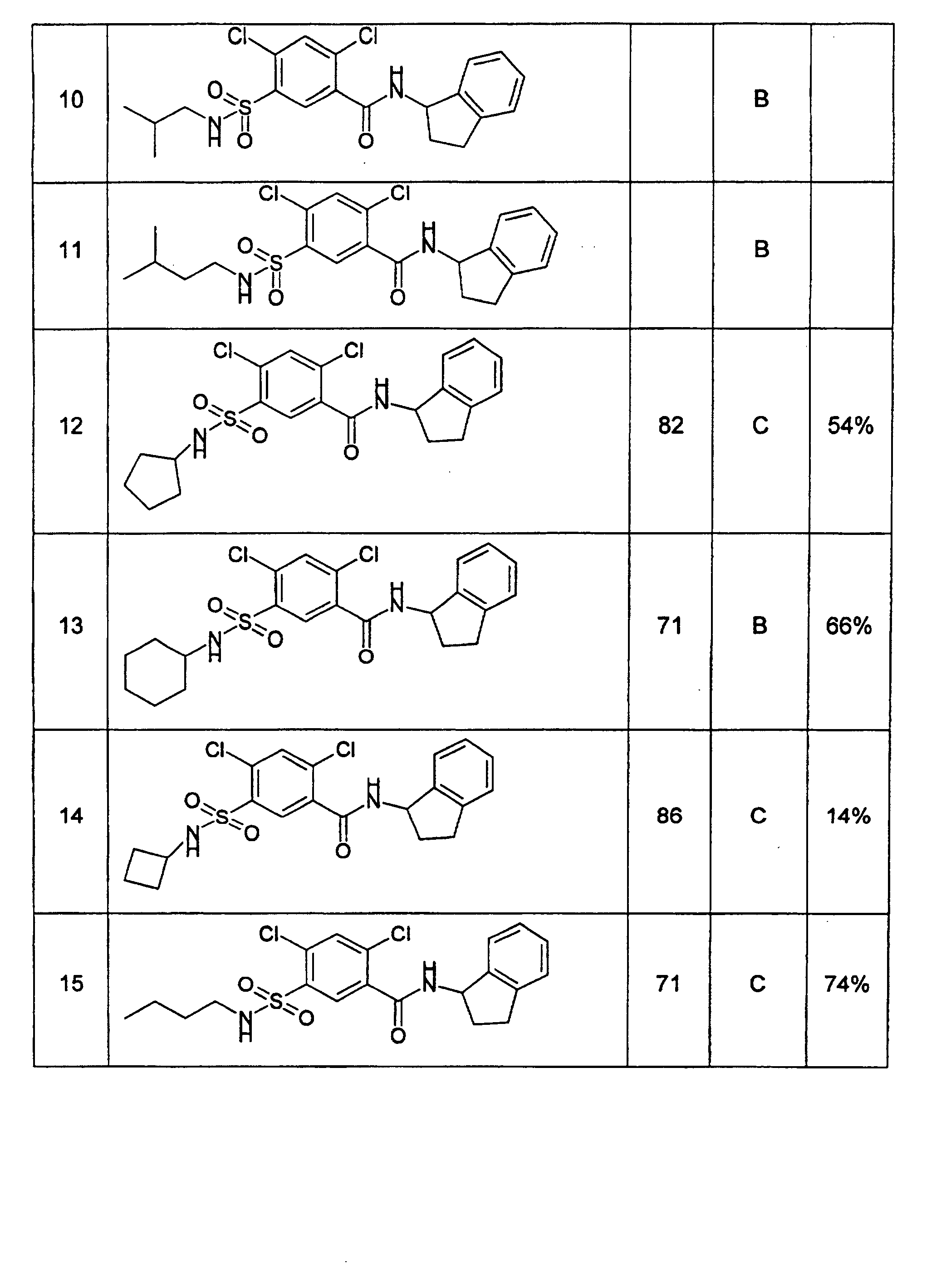

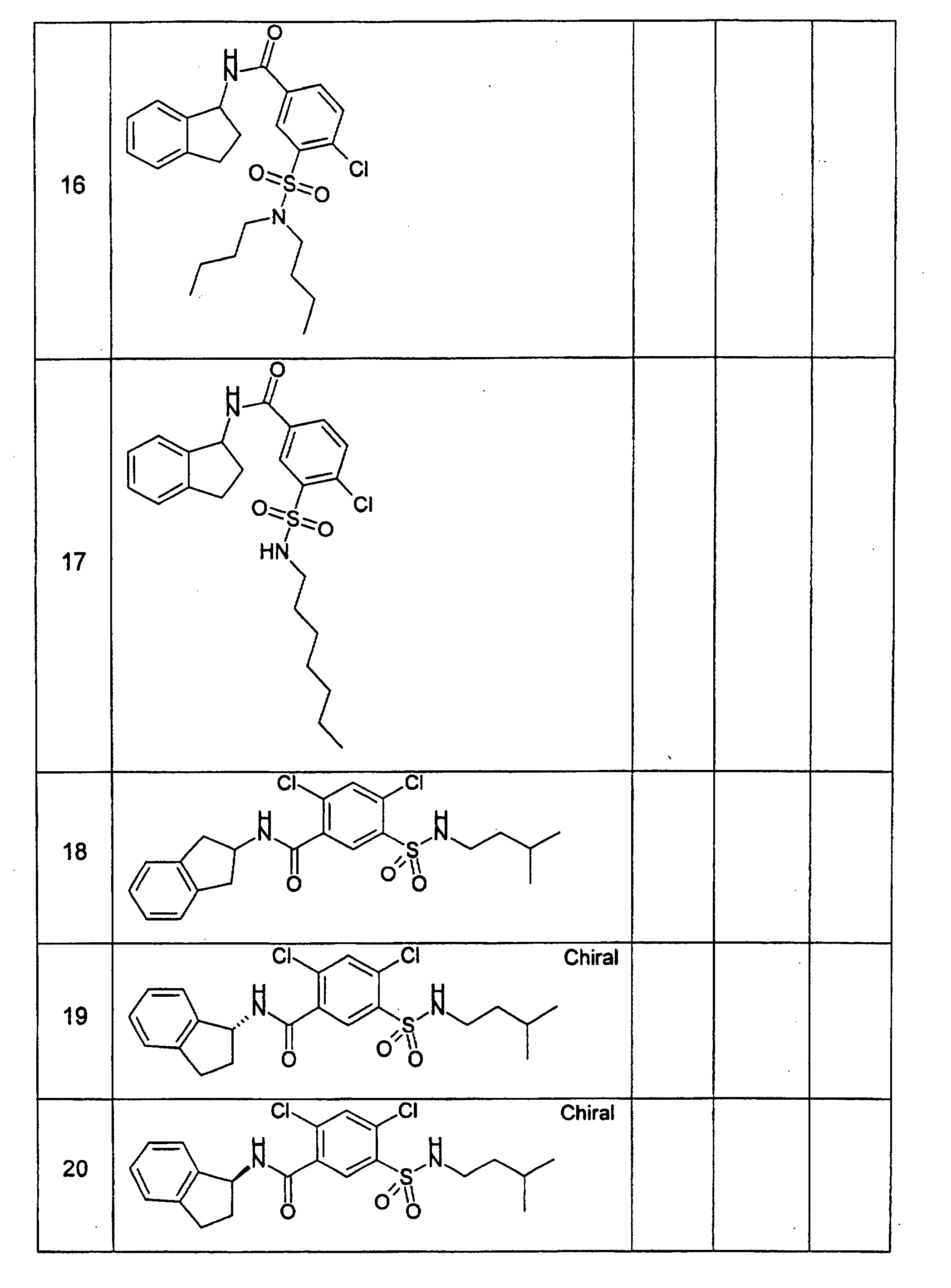

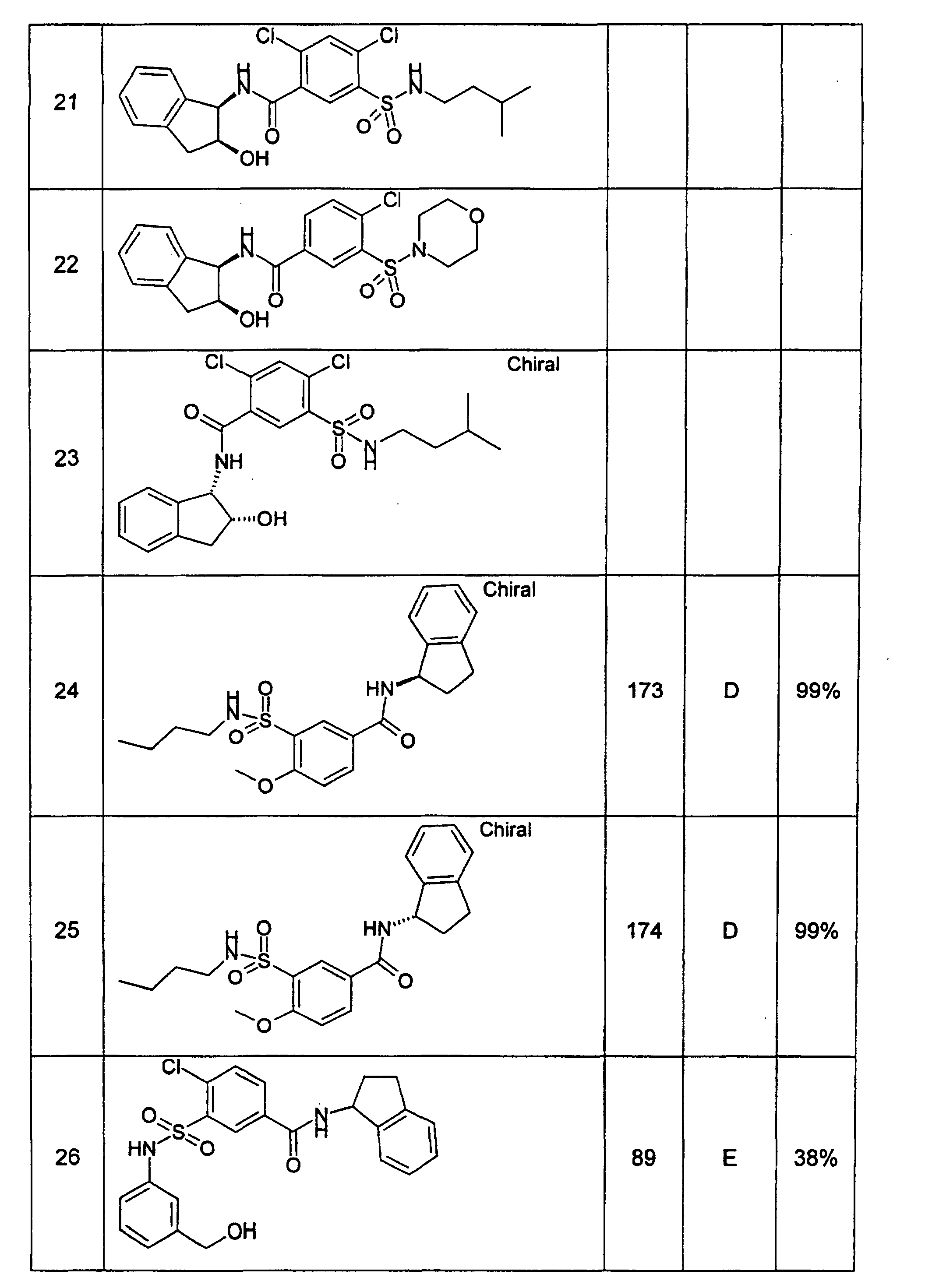

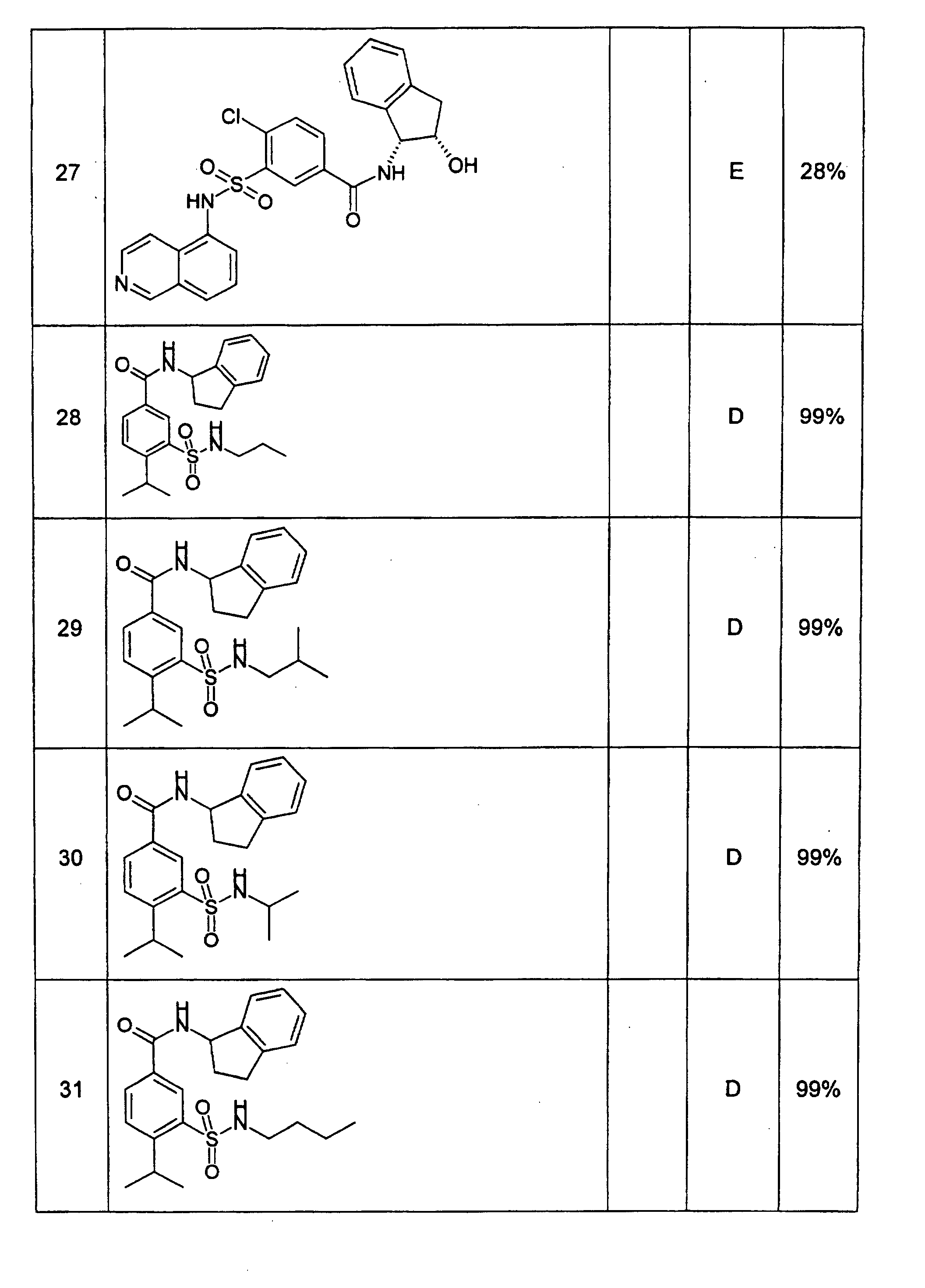

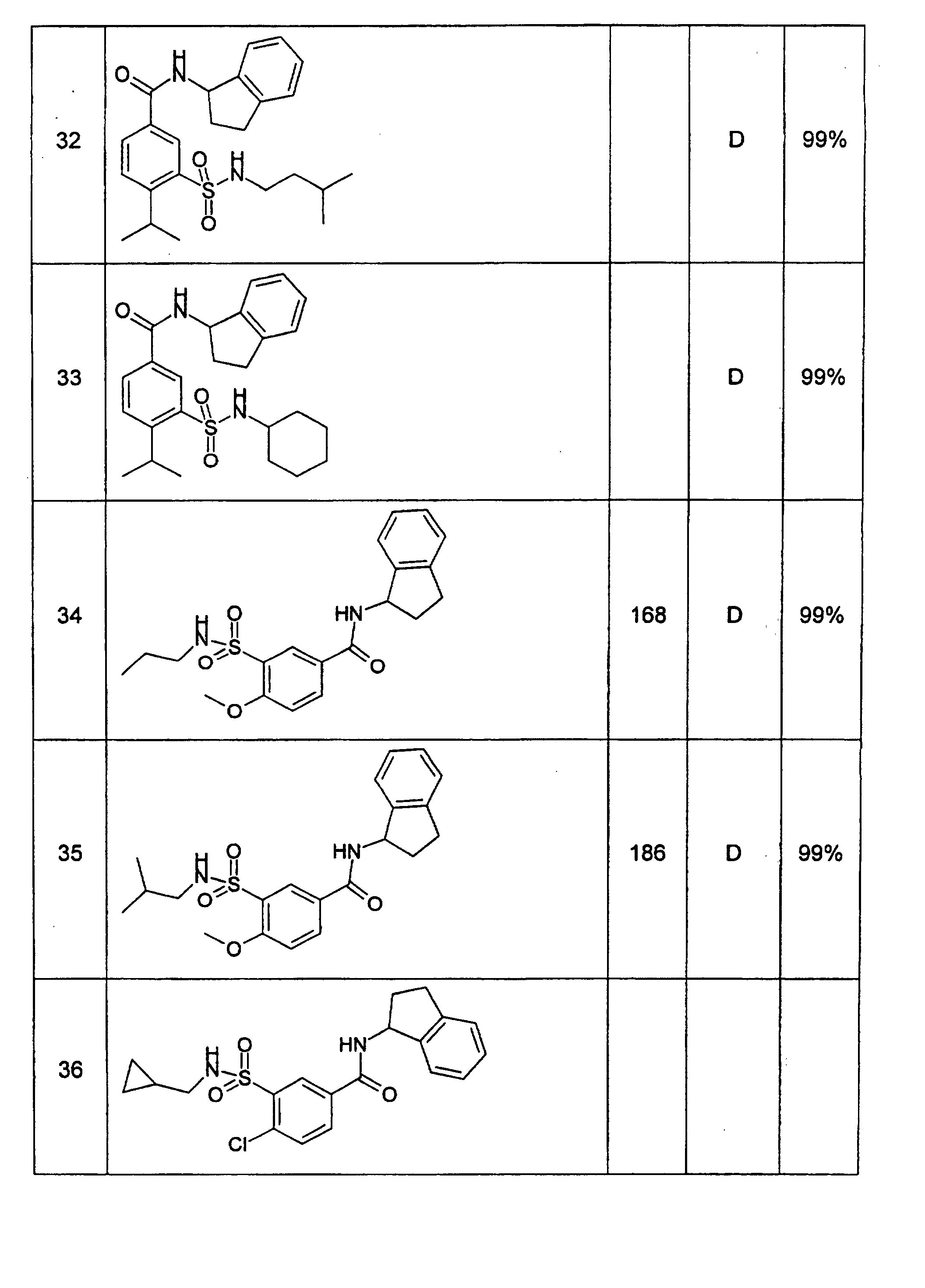

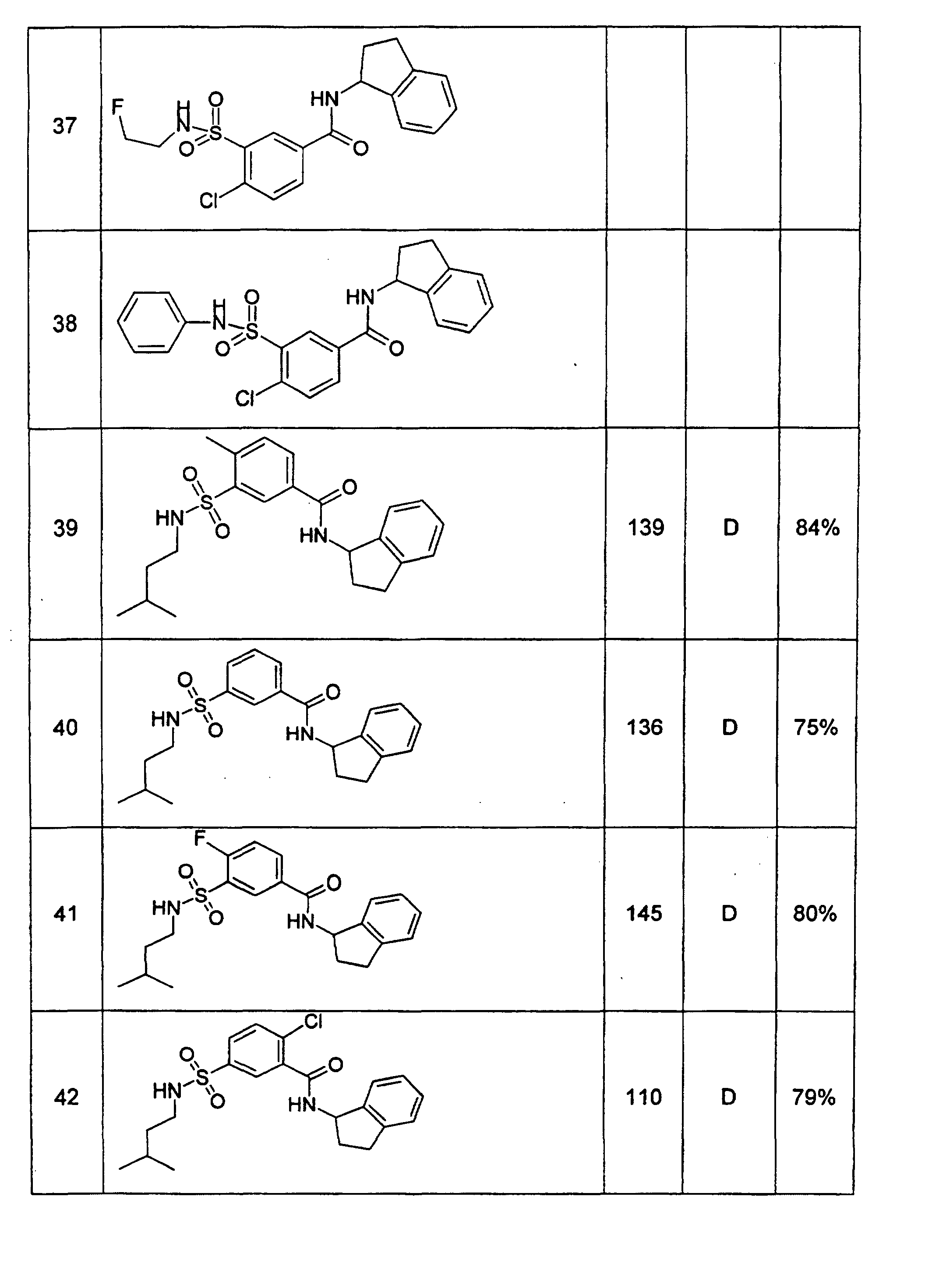

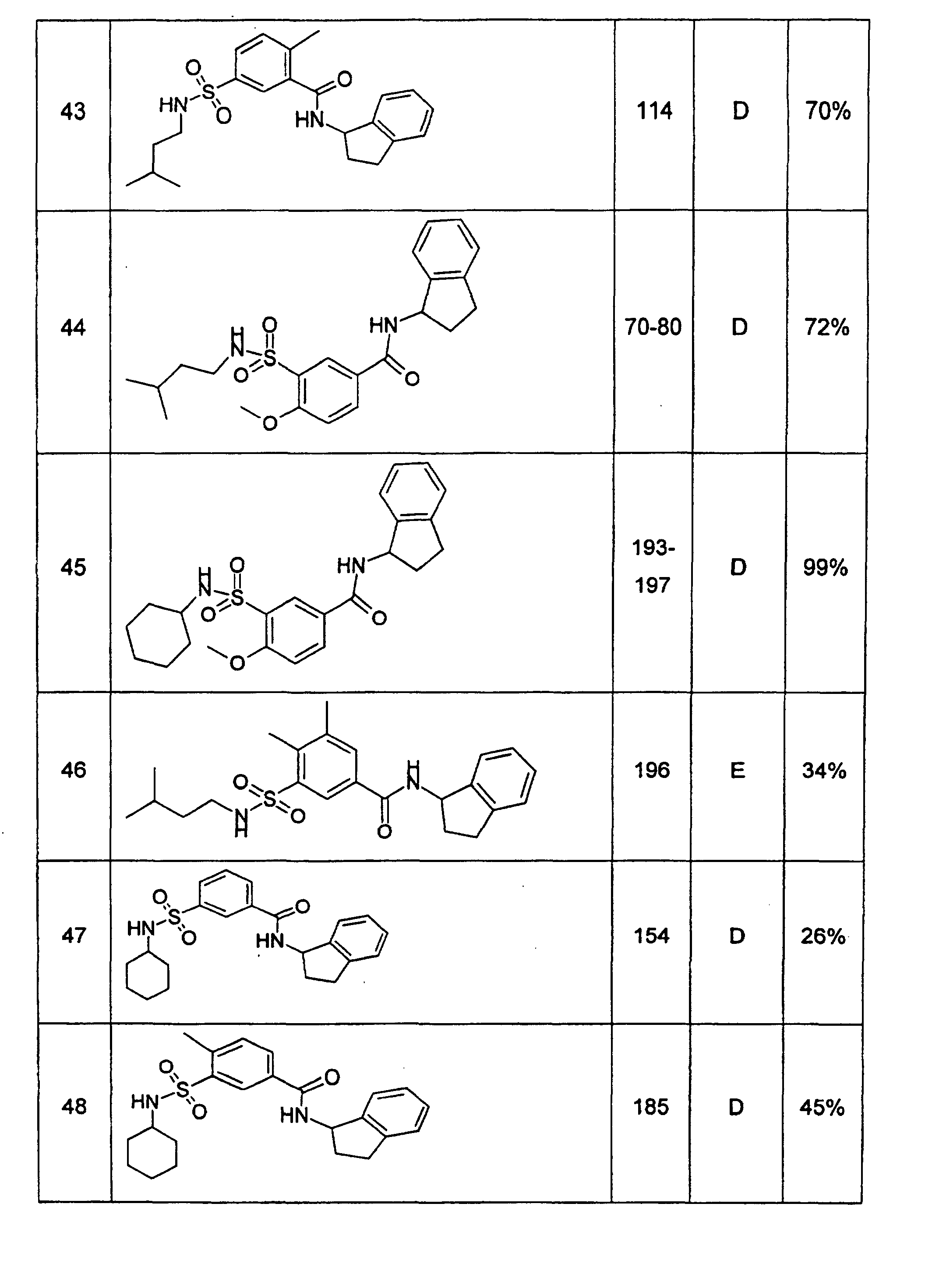

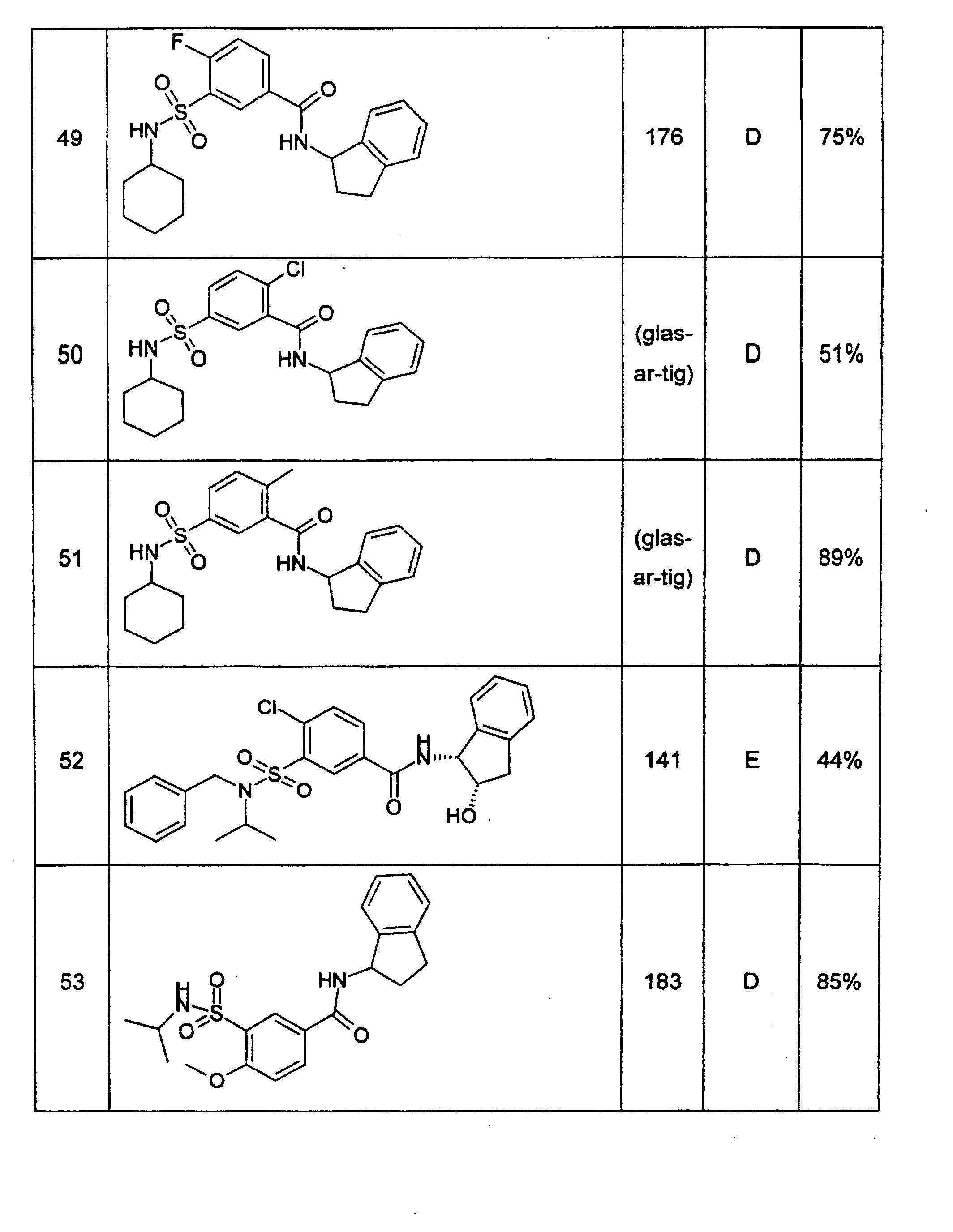

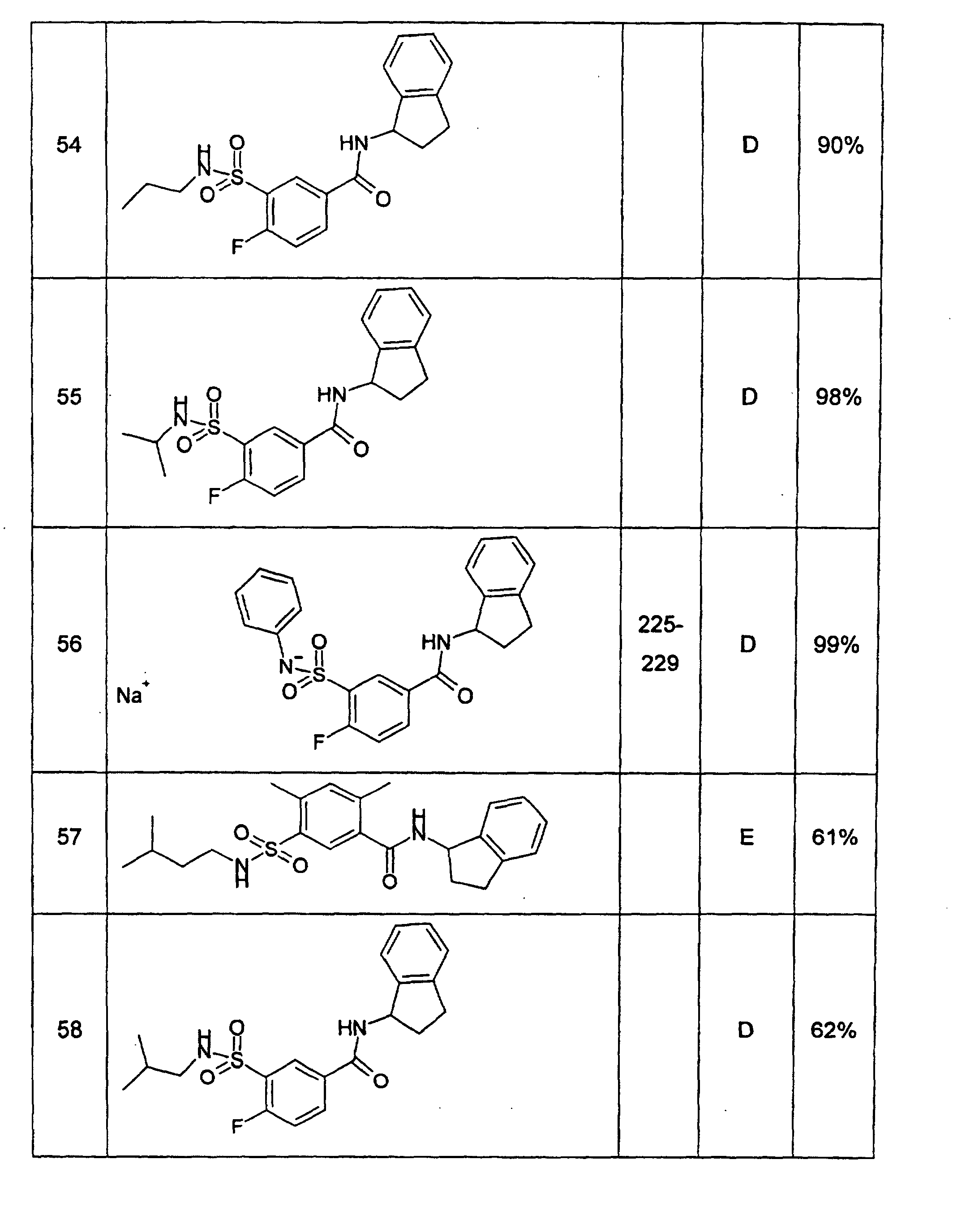

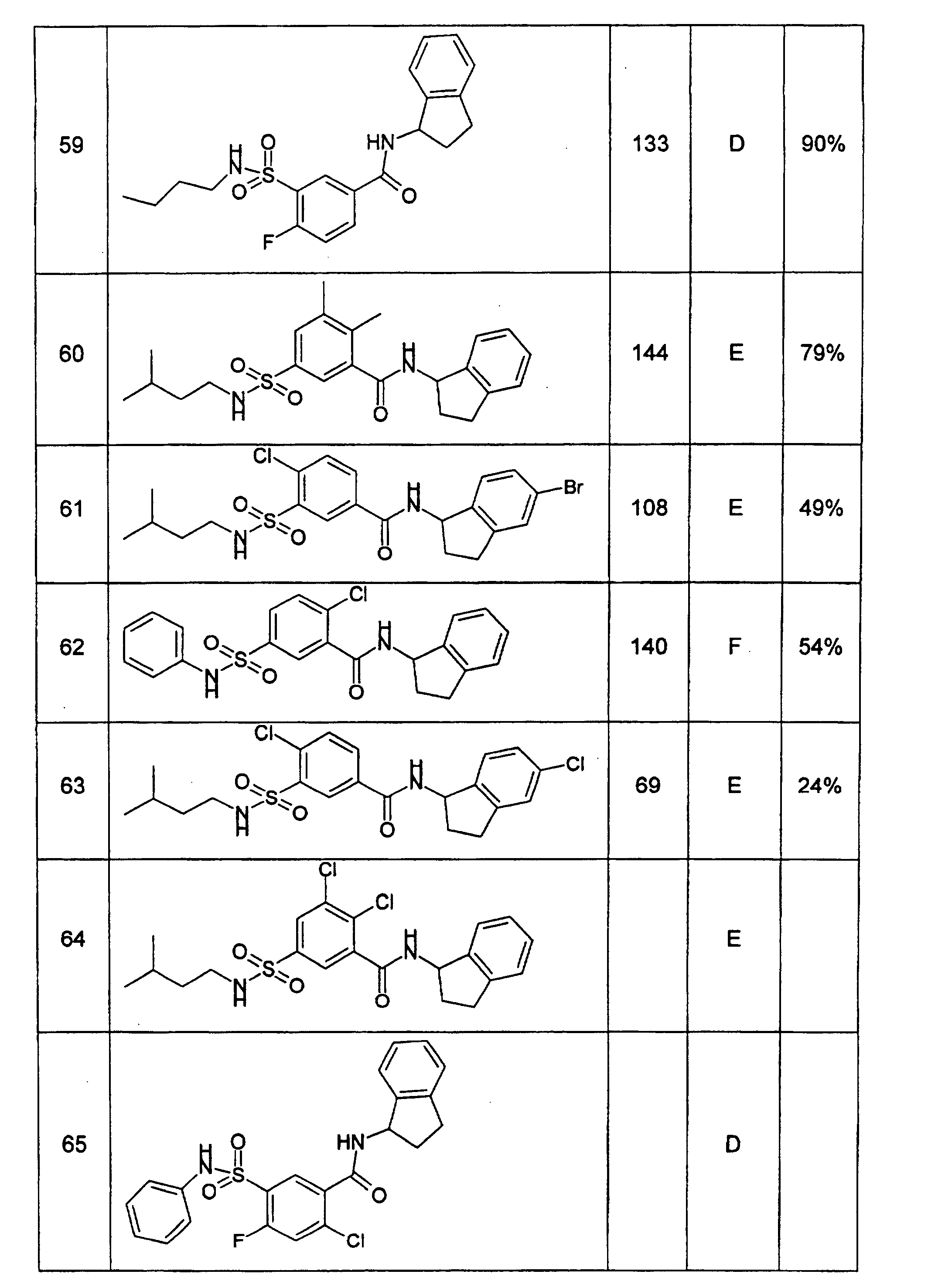

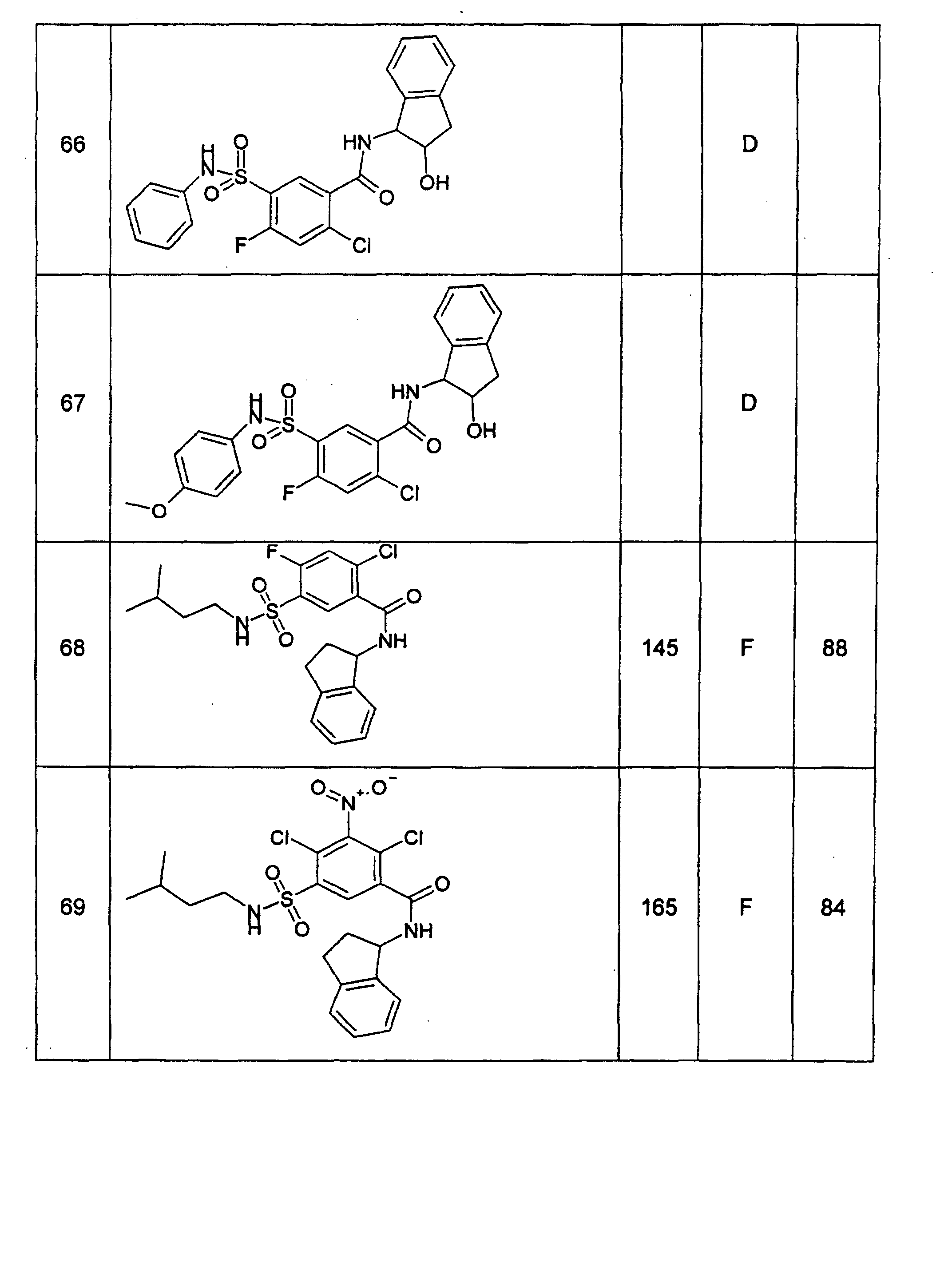

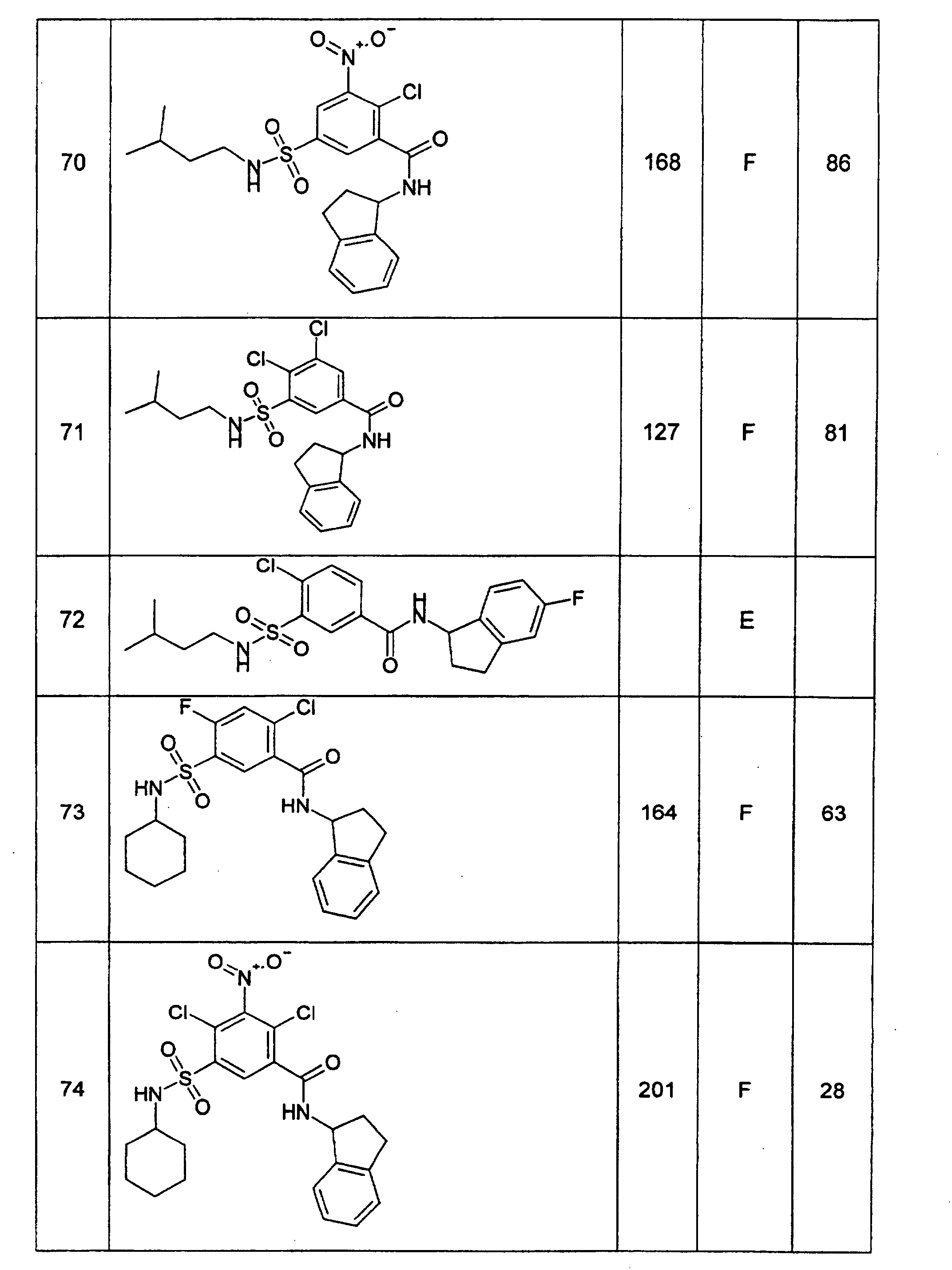

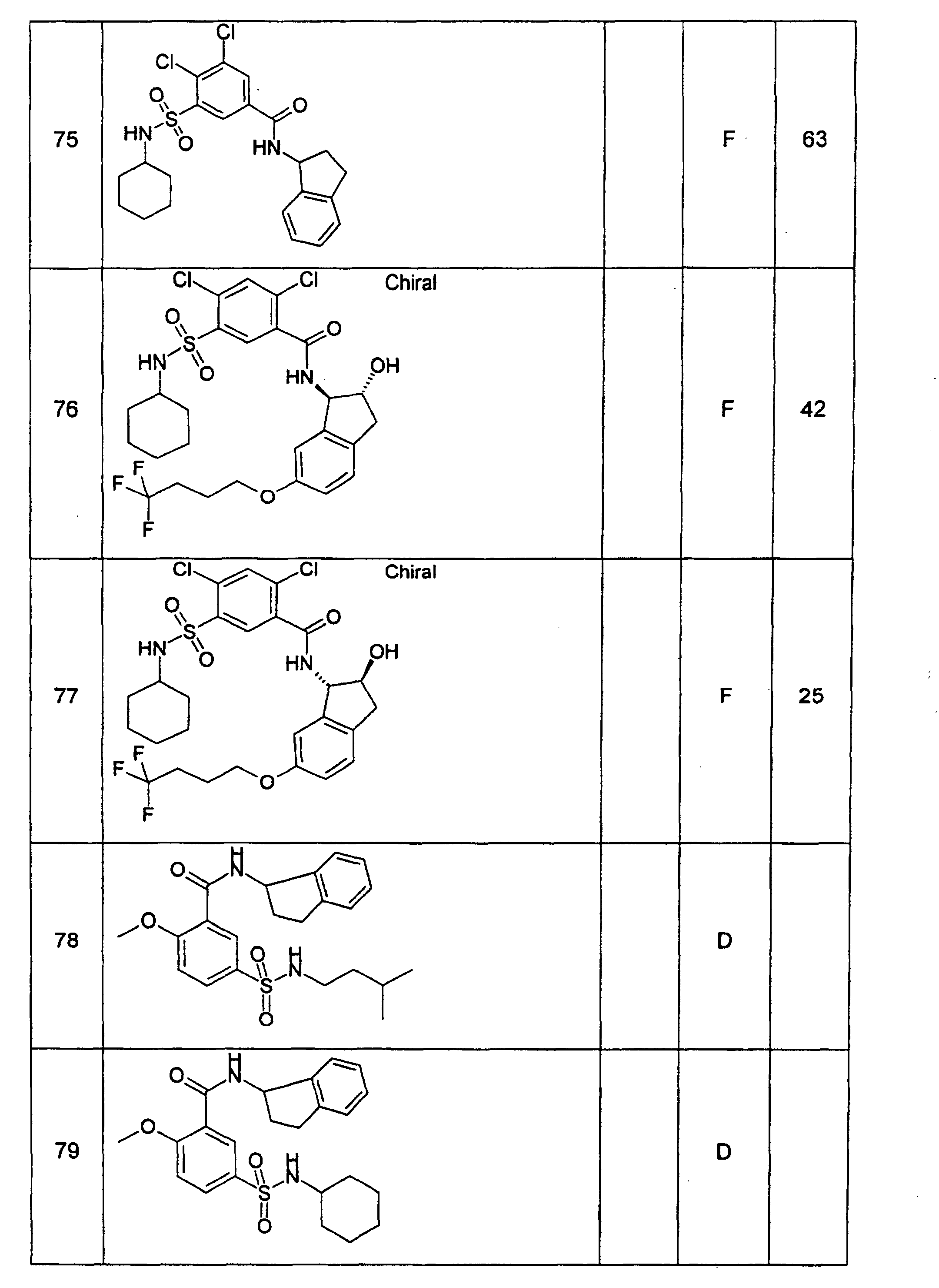

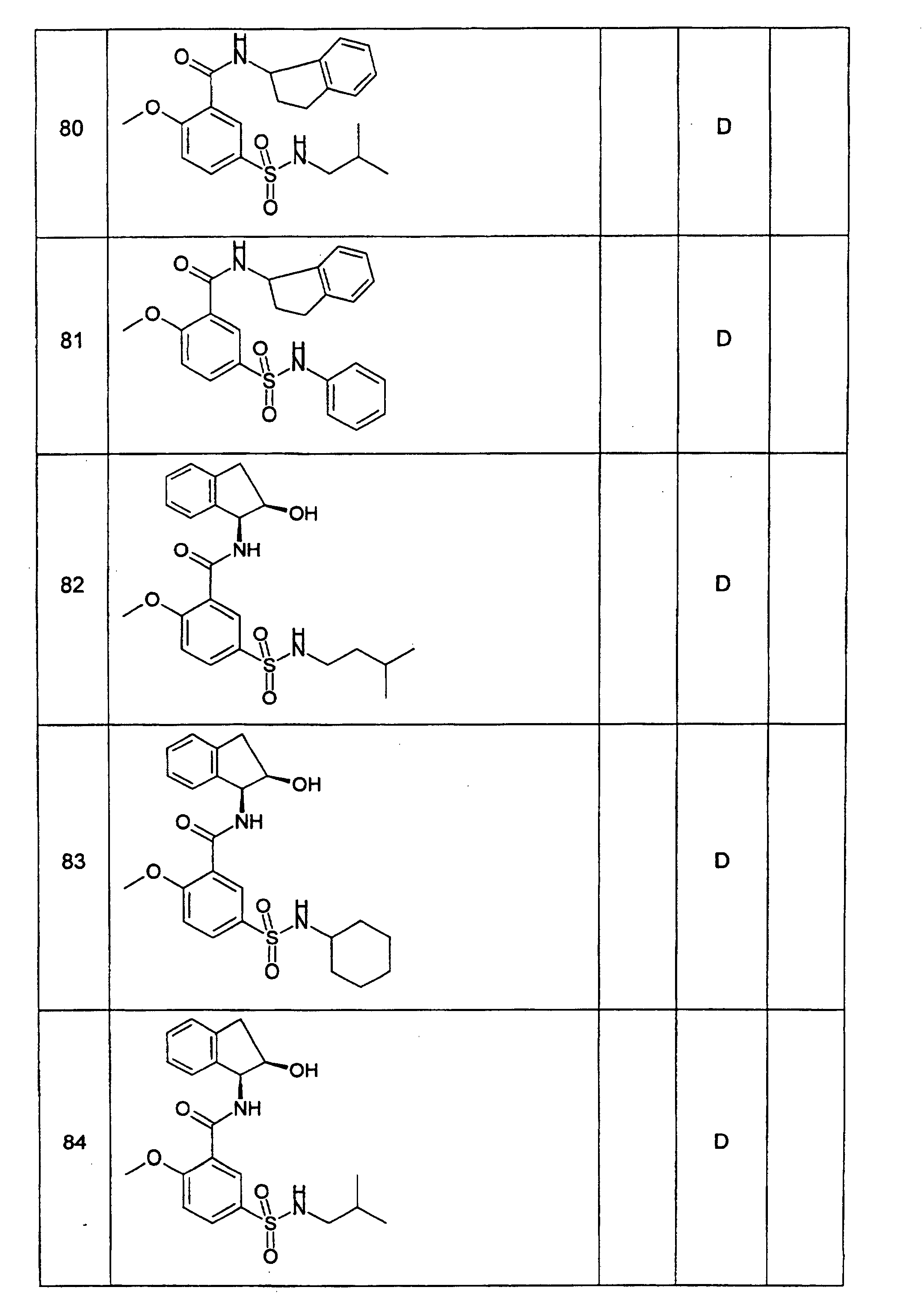

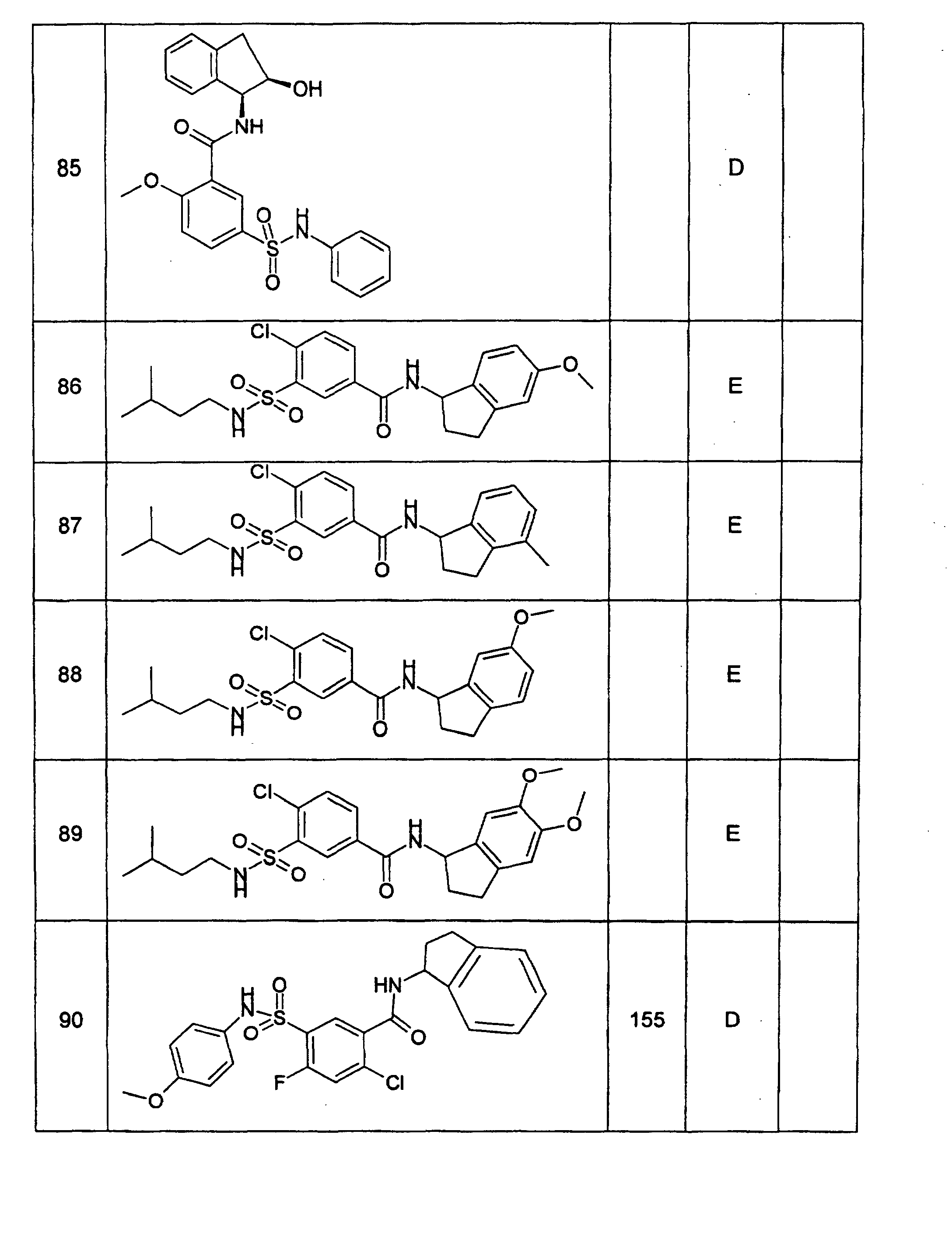

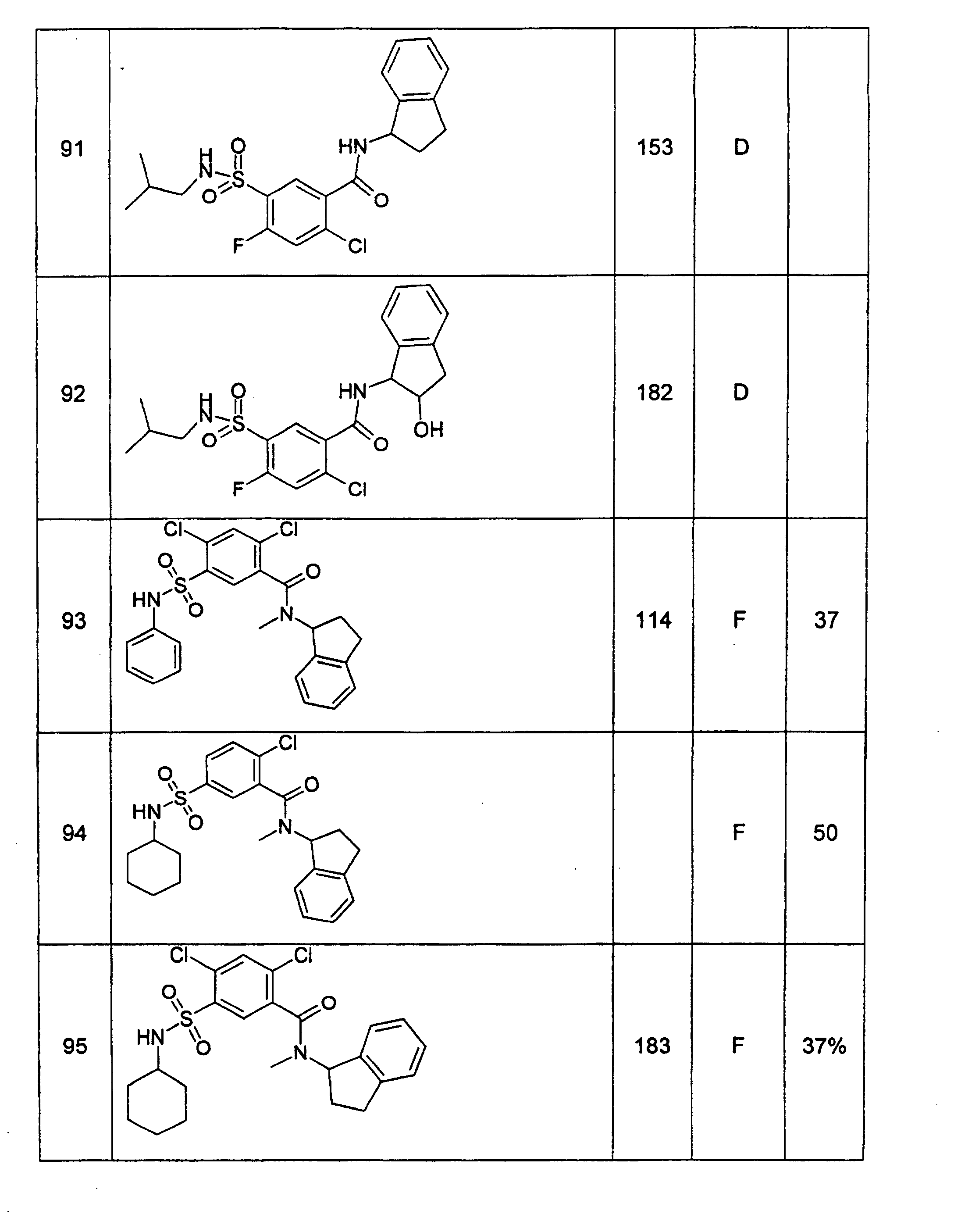

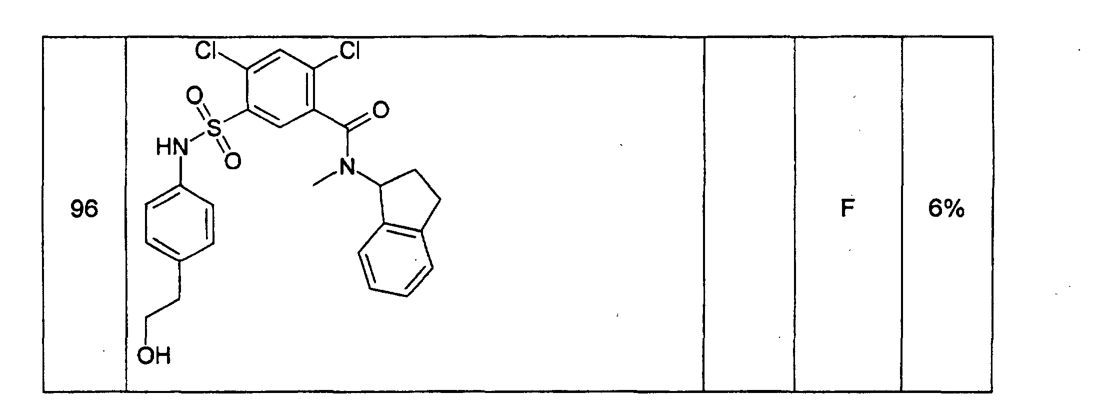

### Beispiel 97:

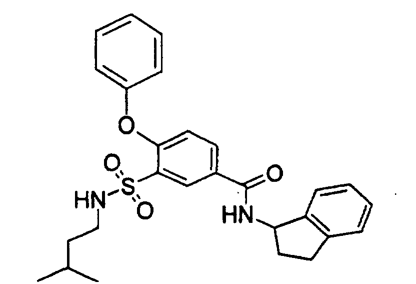

Eine Lösung aus 100 mg 4-Fluor-N-indan-1-yl-3-(3-methylbutylsulfamoyl)-benzamid (Beispiel 41), 26 mg Phenol und 100 mg Kaliumcarbonat in 4 ml N,N-Dimethylacetamid wurde 6 h auf 100°C erhitzt. Nach Zugabe von Wasser und Absaugen des ausgefällten Produktes wurden 82 mg 4-Phenoxy-N-indan-1-yl-3-(3-methylbutylsulfamoyl)-benzamid erhalten; Fp. 73°C.

### Beispiel 98:

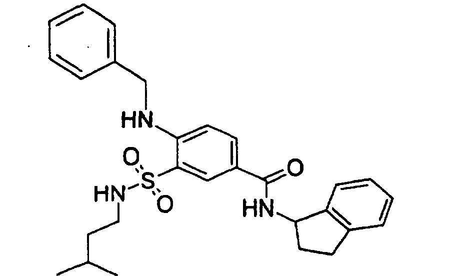

Eine Lösung aus 100 mg 4-Fluor-N-indan-1-yl-3-(3-methylbutylsulfamoyl)-benzamid (Beispiel 41), 29 mg Benzylamin und 100 mg Kaliumcarbonat in 4 ml N,N-Dimethylacetamid wurde 6 h auf 100°C erhitzt. Nach Zugabe von Wasser, Extraktion mit EE und chromatographischer Trennung wurden 33 mg 4-Benzylamino-N-indan-1-yl-3-(3-methyl-butylsulfamoyl)-benzamid erhalten.

### Beispiel 99:

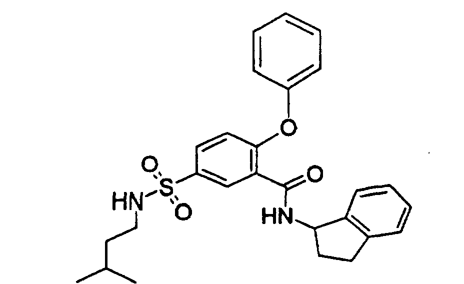

Aus 100 mg 2-Chlor-N-indan-1-yl-5-(3-methyl-butylsulfamoyl)-benzamid (Beispiel 42) wurden analog Beispiel 97 75 mg 2-Phenoxy-N-indan-1-yl-5-(3-methyl-butylsulfamoyl)-benzamid erhalten.

### Pharmakologische Untersuchungen

Kv1.5-Kanäle aus dem Menschen wurden in *Xenopus* Oozyten expremiert. Hierfür wurden zuerst Oozyten aus *Xenopus laevis* isoliert und defollikuliert. Anschließend wurde in diese Oozyten in vitro synthetisierte Kv1.5 kodierende RNA injiziert. Nach 1 - 7 Tagen Kv1.5-Proteinexpression wurden an den Oozyten mit der Zwei-Mikroelektroden Voltage-Clamp Technik Kv1.5-Ströme gemessen. Die Kv1.5-Kanäle wurden hierbei in der Regel mit 500 ms dauernden Spannungssprüngen auf 0 mV und 40 mV aktiviert. Das Bad wurde mit einer Lösung der nachfolgenden Zusammensetzung durchspült: NaCI 96 mM, KCl 2 mM, CaCl₂ 1,8 mM, MgCl₂ 1 mM, HEPES 5 mM (titriert mit NaOH auf pH 7,4). Diese Experimente wurden bei Raumtemperatur durchgeführt. Zur Datenerhebung und Analyse wurden eingesetzt: Geneclamp Verstärker (Axon Instruments, Foster City, USA) und MacLab D/A-Umwandler und Software (ADInstruments, Castle Hill, Australia). Die erfindungsgemäßen Substanzen wurden getestet, indem sie in unterschiedlichen Konzentrationen der Badlösung zugefügt wurden. Die Effekte der Substanzen wurden als prozentuale Inhibition des Kv1.5-Kontrollstromes berechnet, der erhalten wurde, wenn der Lösung keine Substanz zugesetzt wurde. Die Daten wurden anschließend mit der Hill-Gleichung extrapoliert, um die Hemmkonzentrationen IC₅₀ für die jeweiligen Substanzen zu bestimmen.

### Auf diese Weise wurden für die nachfolgend aufgeführten Verbindungen folgende IC₅₀-Werte bestimmt:

| Verbindung | IC₅₀ [µM] |
|---|---|
| Beispiel 1 | >> 10 |
| Beispiel 3 | 7,7 |
| Beispiel 10 | 4,8 |
| Beispiel 11 | 2,8 |
| Beispiel 13 | 3,9 |
| Beispiel 14 | 4,2 |
| Beispiel 19 | ∼ 3 |
| Beispiel 20 | ∼3 |
| Beispiel 43 | 5,3 |
| Beispiel 51 | 7,1 |
| Beispiel 57 | 8,8 |
| Beispiel 60 | 6,9 |
| Beispiel 62 | 6,3 |
| Beispiel 73 | 6,2 |
| Beispiel 88 | 8,7 |
| Beispiel 89 | 5,6 |
| Beispiel 90 | 2,8 |
| Beispiel 94 | 2,2 |

## Patentansprüche

1. Verbindungen der Formel I, 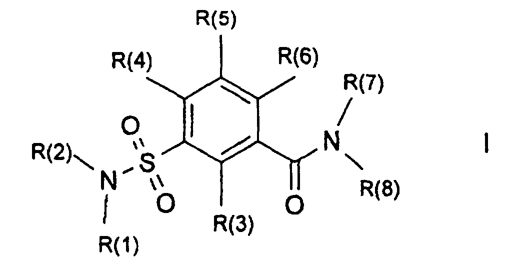 in der R(8) entweder für einen 1-Indanylrest der Formel II oder für einen 2-Indanylrest der Formel III steht, 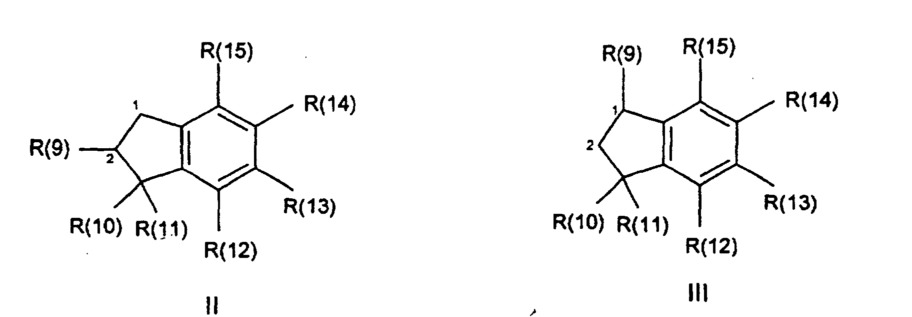 und worin bedeuten:
R(1) und R(2)
unabhängig voneinander R(20)-CᵣH₂ᵣ,
wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(21)- oder -CONR(21);
R(21) Wasserstoff oder Alkyl mit 1, 2 oder 3 C-Atomen;
R(20) H, CH₃, CH₂F, CHF₂, CF₃, C₂F₅, C₃F₇, Cycloalkyl mit 3, 4, 5, 6, 7 oder 8 C-Atomen, NR(22)R(23), -CONR(22)R(23), -OR(24), -COOR(24), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Hydroxymethyl, Hydroxyethyl,
Methoxy, Dimethylamino, Sulfamoyl, Methylsulfonyl und Methylsulfonylamino;
R(22) und R(23)
unabhängig voneinander Wasserstoff oder Alkyl mit 1, 2, 3 oder 4 C-Atomen;
oder R(22) und R(23)
gemeinsam eine Kette von 4 oder 5 Methylengruppen, von denen eine CH₂-Gruppe durch -O-, -S-, -NH-, -N(Methyl)-oder -N(Benzyl)- ersetzt sein kann;
R(24) Wasserstoff oder Alkyl mit 1, 2 oder 3 C-Atomen;
r Null, 1, 2, 3, 4, 5, 6, 7 oder 8;
oder
R(1) und R(2)
gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
R(3), R(4), R(5) und R(6)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, NO₂, OR(25) oder NR(26)R(27);
R(25) Wasserstoff, Alkyl mit 1, 2, 3 oder 4 C-Atomen, ein fluorierter Alkylrest der Formel -CₓH₂ₓCF_{y}H_{3-y} oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sutfamoyl, Methylsulfonyl und Methylsulfonylamino;
x 0, 1, 2 oder 3;
y 1, 2 oder 3;
R(26) und R(27)
unabhängig voneinander Wasserstoff oder Alkyl mit 1, 2, 3 oder 4 C-Atomen;
oder
R(26) und R(27)
gemeinsam eine Kette von 4 oder 5 Methylengruppen, von denen eine CH₂-Gruppe durch -O-, -S-, -NH-, -N(Methyl)- oder -N(Benzyl)-ersetzt sein kann;
R(7) Wasserstoff oder Alkyl mit 1, 2, 3 oder 4 C-Atomen;
R(9) Wasserstoff, OR(28) oder OCOR(28);
R(28) Wasserstoff oder Alkyl mit 1, 2, 3 oder 4 C-Atomen;
R(10) und R(11)
unabhängig voneinander Wasserstoff oder Alkyl mit 1, 2, 3 oder 4 C-Atomen;
R(12), R(13), R(14) und R(15)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(29), Phenyl, Thienyl, Furyl oder ein N-haltiger Heterocyclus mit 1, 2, 3, 4, 5, 6, 7, 8 oder 9 C-Atomen,
wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-, -O-SO₂-, -SO₂NR(30)-, -CONR(30)- oder -NR(30)CO-, wobei die Verknüpfung mit dem Grundgerüst jeweils über das links stehende Atom erfolgt; R(30) Wasserstoff oder Alkyl mit 1, 2 oder 3 C-Atomen; s Null, 1, 2, 3, 4, 5 oder 6;
R(29) Wasserstoff, Methyl, CF₃, C₂F₅, C₃F₇, Cycloalkyl mit 3, 4, 5, 6, 7 oder 8 C-Atomen, -OR(31), -COOR(31), -NR(32)R(33), -CONR(32)R(33), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(31) Wasserstoff oder Alkyl mit 1, 2 oder 3 C-Atomen;
R(32) und R(33)
unabhängig voneinander Wasserstoff oder Alkyl mit 1, 2, 3 oder 4 C-Atomen;
oder
R(32) und R(33)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
sowie ihre physiologisch verträglichen Salze.

2. Verbindungen der Formel I nach Anspruch 1, wobei jedoch mindestens einer der Reste R(1) oder R(2) eine andere Bedeutung als Wasserstoff hat.

3. Verbindungen der Formel I nach Ansprüchen 1 oder 2, in der R(8) entweder für einen 1-Indanylrest der Formel II oder für einen 2-Indanylrest der Formel III steht und worin bedeuten:
R(1) Wasserstoff;
R(2) R(20)-CᵣH₂ᵣ,
wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(21)- oder -CONR(21);
R(21) Wasserstoff oder Alkyl mit 1, 2 oder 3 C-Atomen;
R(20) CH₃, CH₂F, CHF₂, CF₃, C₂F₅, C₃F₇, Cycloalkyl mit 3, 4, 5, 6, 7 oder 8 C-Atomen, NR(22)R(23), -CONR(22)R(23), -OR(24), -COOR(24), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Hydroxymethyl, Hydroxyethyl, Methoxy, Dimethylamino, Sulfamoyl, Methylsulfonyl und Methylsulfonylamino;
R(22) und R(23)
unabhängig voneinander Wasserstoff oder Alkyl mit 1, 2, 3 oder 4 C-Atomen;
oder
R(22) und R(23)
gemeinsam eine Kette von 4 oder 5 Methylengruppen, von denen eine CH₂-Gruppe durch -O-, -S-, -NH-, -N(Methyl)-oder -N(Benzyl)- ersetzt sein kann;
R(24) Wasserstoff oder Alkyl mit 1, 2 oder 3 C-Atomen;
r Null, 1, 2, 3, 4, 5, 6, 7 oder 8;
R(3), R(4), R(5) und R(6)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, NO₂, OR(25) oder NR(26)R(27);
R(25) Wasserstoff, Alkyl mit 1, 2, 3 oder 4 C-Atomen, ein fluorierter Alkylrest der Formel -CₓH₂ₓCF_{y}H_{3-y} oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
x 0, 1, 2 oder 3;
y 1, 2 oder 3;
R(26) und R(27)
unabhängig voneinander Wasserstoff oder Alkyl mit 1, 2, 3 oder 4 C-Atomen;
oder
R(26) und R(27)
gemeinsam eine Kette von 4 oder 5 Methylengruppen, von denen eine CH₂-Gruppe durch -O-, -S-, -NH-, -N(Methyl)- oder -N(Benzyl)-ersetzt sein kann;
R(7) Wasserstoff oder Alkyl mit 1, 2, 3 oder 4 C-Atomen;
R(9) Wasserstoff, OR(28) oder OCOR(28);
R(28) Wasserstoff oder Alkyl mit 1, 2, 3 oder 4 C-Atomen;
R(10) und R(11)
unabhängig voneinander Wasserstoff oder Alkyl mit 1, 2, 3 oder 4 C-Atomen;
R(12), R(13), R(14) und R(15)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(29), Phenyl, Thienyl, Furyl oder ein N-haltiger Heterocyclus mit 1, 2, 3, 4, 5, 6, 7, 8 oder 9 C-Atomen,
wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-, -O-SO₂-, -SO₂NR(30)-, -CONR(30)- oder -NR(30)CO-, wobei die Verknüpfung mit dem Grundgerüst jeweils über das links stehende Atom erfolgt; R(30) Wasserstoff oder Alkyl mit 1, 2 oder 3 C-Atomen;
s Null, 1, 2, 3, 4, 5 oder 6;
R(29) Wasserstoff, Methyl, CF₃, C₂F₅, C₃F₇, Cycloalkyl mit 3, 4, 5, 6, 7 oder 8 C-Atomen, -OR(31), -COOR(31), -NR(32)R(33), -CONR(32)R(33), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
s R(31) Wasserstoff oder Alkyl mit 1, 2 oder 3 C-Atomen;
R(32) und R(33)
unabhängig voneinander Wasserstoff oder Alkyl mit 1, 2, 3 oder 4 C-Atomen;
oder
R(32) und R(33)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
sowie ihre physiologisch verträglichen Salze.

4. Verbindungen der Formel I nach einem oder mehreren den Ansprüche 1 bis 3, in der R(8) für einen 1-Indanylrest der Formel II steht, also Verbindungen der Formel l a 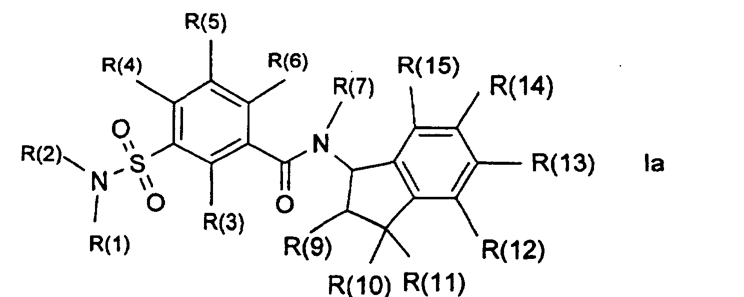 worin bedeuten:
R(1) Wasserstoff;
R(2) R(20)-CYᵣH₂ᵣ,
wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(21)- oder -CONR(21);
R(21) Wasserstoff oder Alkyl mit 1, 2 oder 3 C-Atomen;
R(20) CH₃, CH₂F, CHF₂, CF₃, C₂F₅, C₃F₇, Cycloalkyl mit 3, 4, 5, 6, 7 oder 8 C-Atomen, NR(22)R(23), -CONR(22)R(23), -OR(24), -COOR(24), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Hydroxymethyl, Hydroxyethyl, Methoxy, Dimethylamino, Sulfamoyl, Methylsulfonyl und Methylsulfonylamino;
R(22) und R(23)
unabhängig voneinander Wasserstoff oder Alkyl mit 1, 2, 3 oder 4 C-Atomen;
oder
R(22) und R(23)
gemeinsam eine Kette von 4 oder 5 Methylengruppen, von denen eine CH₂-Gruppe durch -O-, -S-, -NH-, -N(Methyl)-oder -N(Benzyl)- ersetzt sein kann;
R(24) Wasserstoff oder Alkyl mit 1, 2 oder 3 C-Atomen;
r Null, 1, 2, 3, 4, oder 5;
R(3), R(4), R(5) und R(6)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, NO₂ oder OR(25);
R(25) Wasserstoff, Alkyl mit 1, 2, 3 oder 4 C-Atomen, ein fluorierter Alkylrest der Formel -CₓH₂ₓCF_{y}H_{3-y} oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
x 0, 1, 2 oder 3;
y 1, 2 oder 3;
R(7) Wasserstoff oder Alkyl mit 1, 2, 3 oder 4 C-Atomen;
R(9) Wasserstoff, OR(28) oder OCOR(28);
R(28) Wasserstoff oder Alkyl mit 1, 2, 3 oder 4 C-Atomen;
R(10) und R(11)
unabhängig voneinander Wasserstoff oder Alkyl mit 1, 2, 3 oder 4 C-Atomen;
R(12), R(13), R(14) und R(15) unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -NO₂, -Y-CₛH₂ₛ-R(29), Phenyl, Thienyl, Furyl oder ein N-haltiger Heterocyclus mit 1, 2, 3, 4, 5, 6, 7, 8 oder 9 C-Atomen,
wobei Phenyl, Thienyl, Furyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-, -O-SO₂-, -SO₂NR(30)-, -CONR(30)- oder -NR(30)CO-, wobei die Verknüpfung mit dem Grundgerüst jeweils über das links stehende Atom erfolgt; R(30) Wasserstoff oder Alkyl mit 1, 2 oder 3 C-Atomen;
s Null, 1, 2, 3, 4, 5 oder 6;
R(29) Wasserstoff, Methyl, CF₃, C₂F₅, C₃F₇, Cycloalkyl mit 3, 4, 5, 6, 7 oder 8 C-Atomen, -OR(31), -COOR(31), -NR(32)R(33), -CONR(32)R(33), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(31) Wasserstoff oder Alkyl mit 1, 2 oder 3 C-Atomen;
R(32) und R(33)
unabhängig voneinander Wasserstoff oder Alkyl mit 1, 2, 3 oder 4 C-Atomen;
oder
R(32) und R(33)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
sowie ihre physiologisch verträglichen Salze.

5. Verbindungen der Formel I a nach einem oder mehreren der Ansprüche 1 bis 4, worin bedeuten:
R(1) Wasserstoff;
R(2) R(20)-CᵣH₂ᵣ;
R(20) CH₃, CH₂F, CHF₂, CF₃, Cycloalkyl mit 3, 4, 5, 6, 7 oder 8 C-Atomen, -CONR(22)R(23), -OR(24), -COOR(24) oder Phenyl,
das unsubstituiert ist oder substituiert mit 1 oder 2 Substituenten, ausgewählt aus der Gruppe bestehend aus F, Cl, Br, CF₃, NO₂, CN, OH, Methyl, Ethyl, Hydroxymethyl, Hydroxyethyl, Methoxy, Dimethylamino, Sulfamoyl, Methylsulfonyl und Methylsulfonylamino;
R(22) und R(23)
unabhängig voneinander Wasserstoff oder Alkyl mit 1, 2, 3 oder 4 C-Atomen;
oder
R(22) und R(23)
gemeinsam eine Kette von 4 oder 5 Methylengruppen, von denen eine CH₂-Gruppe durch -O-, -S-, -NH-, -N(Methyl)-oder -N(Benzyl)- ersetzt sein kann;
R(24) Wasserstoff oder Alkyl mit 1, 2 oder 3 C-Atomen;
r Null, 1, 2, 3, 4, oder 5;
R(3), R(4), R(5) und R(6)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, NO₂ oder OR(25);
R(25) Wasserstoff, Alkyl mit 1, 2, 3 oder 4 C-Atomen, ein fluorierter Alkylrest der Formel -CₓH₂ₓCF_{y}H_{3-y} oder Phenyl, das unsubstituiert ist oder substituiert mit 1 oder 2 Substituenten, ausgewählt aus der Gruppe bestehend aus F, Cl, Br, CF₃, NO₂, CN, OH, Methyl, Ethyl, Methoxy, Dimethylamino, Sulfamoyl, Methylsulfonyl und Methylsulfonylamino;
x 0, 1, 2 oder 3;
y 1, 2 oder 3;
R(7) Wasserstoff oder Alkyl mit 1, 2, 3 oder 4 C-Atomen;
R(9) Wasserstoff oder OR(28);
R(28) Wasserstoff oder Alkyl mit 1, 2, 3 oder 4 C-Atomen;
R(10) und R(11)
unabhängig voneinander Wasserstoff oder Alkyl mit 1, 2, 3 oder 4 C-Atomen;
R(12), R(13), R(14) und R(15)
unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, -NO₂ oder -Y-CₛH₂ₛ-R(29);
Y -O-, -CO-, -CO-O-, -O-CO-, -S-, -SO-, -SO₂-, -O-SO₂-, -SO₂NR(30)-, -CONR(30)- oder -NR(30)CO-, wobei die Verknüpfung mit dem Grundgerüst jeweils über das links stehende Atom erfolgt; R(30) Wasserstoff oder Alkyl mit 1, 2 oder 3 C-Atomen;
s Null, 1, 2, 3, 4 oder 5;
R(29) Wasserstoff, Methyl, CF₃, -OR(31), -COOR(31), -NR(32)R(33), -CONR(32)R(33) oder Phenyl,
das unsubstituiert ist oder substituiert mit 1 oder 2 Substituenten, ausgewählt aus der Gruppe bestehend aus F, Cl, Br, CF₃, NO₂, CN, OH, Methyl, Ethyl, Methoxy, Dimethylamino, Sulfamoyl, Methylsulfonyl und Methylsulfonylamino;
R(31) Wasserstoff oder Alkyl mit 1, 2 oder 3 C-Atomen;
R(32) und R(33)
unabhängig voneinander Wasserstoff oder Alkyl mit 1, 2, 3 oder 4 C-Atomen;
oder
R(32) und R(33)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
sowie ihre physiologisch verträglichen Salze.

6. Verfahren zur Herstellung von Verbindungen der Formel I gemäß einem oder mehreren der Ansprüche 1 bis 5, **dadurch gekennzeichnet, daß** man eine Carbonsäure der Formel IV, 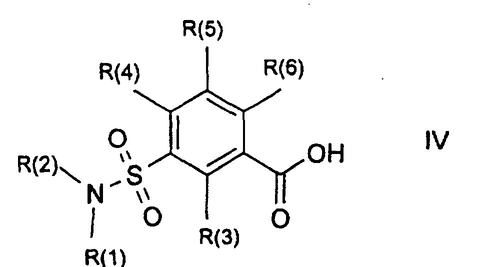 worin R(1), R(2), R(3), R(4), R(5) und R(6) die in den Ansprüchen 1 bis 5 angegebenen Bedeutungen besitzen, in einer Amidierungsreaktion mit einem Amin der Formel V a oder V b umsetzt, 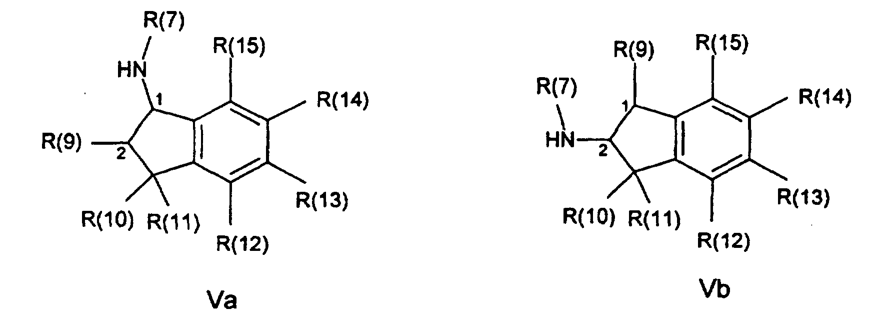 worin R(7), R(9), R(10), R(11), R(12), R(13), R(14) und R(15) die in den Ansprüchen 1 bis 6 angegebenen Bedeutungen besitzen.

7. Verbindungen der Formel I nach einem oder mehreren der Ansprüche 1 bis 5 und ihre physiologisch verträglichen Salze zur Anwendung als Arzneimittel.

8. Pharmazeutische Zubereitung, enthaltend eine wirksame Menge mindestens einer Verbindung der Formel I nach einem oder mehreren der Ansprüche 1 bis 5 und/oder eines physiologisch verträglichen Salzes davon als Wirkstoff, zusammen mit pharmazeutisch annehmbaren Träger- und Zusatzstoffen und gegebenenfalls noch einem oder mehreren anderen pharmakologischen Wirkstoffen.

9. Verwendung einer Verbindung der Formel I nach einem oder mehreren der Ansprüche 1 bis 5 und/oder eines physiologisch verträglichen Salzes davon zur Herstellung eines Medikaments mit K⁺-Kanal-blockierender Wirkung zur Therapie und Prophylaxe von K⁺-Kanal mediierten Krankheiten.

10. Verwendung einer Verbindung der Formel I nach einem oder mehreren der Ansprüche 1 bis 5 und/oder eines physiologisch verträglichen Salzes davon zur Herstellung eines Medikaments zur Therapie oder Prophylaxe von Herzrhythmusstörungen, die durch Aktionspotential-Verlängerung behoben werden können.

11. Verwendung einer Verbindung der Formel I nach einem oder mehreren der Ansprüche 1 bis 5 und/oder eines physiologisch verträglichen Salzes davon zur Herstellung eines Medikaments zur Therapie oder Prophylaxe von Reentry-Arrhythmien.

12. Verwendung einer Verbindung der Formel I nach einem oder mehreren der Ansprüche 1 bis 5 und/oder eines physiologisch verträglichen Salzes davon zur Herstellung eines Medikaments zur Therapie oder Prophylaxe von supraventrikulären Arrhythmien.

13. Verwendung einer Verbindung der Formel I nach einem oder mehreren der Ansprüche 1 bis 5 und/oder eines physiologisch verträglichen Salzes davon zur Herstellung eines Medikaments zur Therapie oder Prophylaxe von atrialer Fibrillation oder atrialem Flattern.

14. Pharmazeutische Zubereitung, enthaltend eine wirksame Menge mindestens einer Verbindung der Formel I nach einem oder mehreren der Ansprüche 1 bis 5 und/oder eines physiologisch verträglichen Salzes davon sowie eines Beta-blockers als Wirkstoffe, zusammen mit pharmazeutisch annehmbaren Träger- und Zusatzstoffen.

15. Pharmazeutische Zubereitung, enthaltend eine wirksame Menge mindestens einer Verbindung der Formel I nach einem oder mehreren der Ansprüche 1 bis 5 und/oder eines physiologisch verträglichen Salzes davon sowie eines IKs-Kanalblockers als Wirkstoffe, zusammen mit pharmazeutisch annehmbaren Trägerund Zusatzstoffen.

## Claims

1. A compound of the formula I 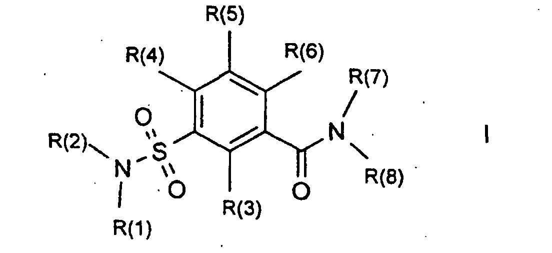 in which R(8) is either a 1-indanyl radical of the formula II or a 2-indanyl radical of the formula III 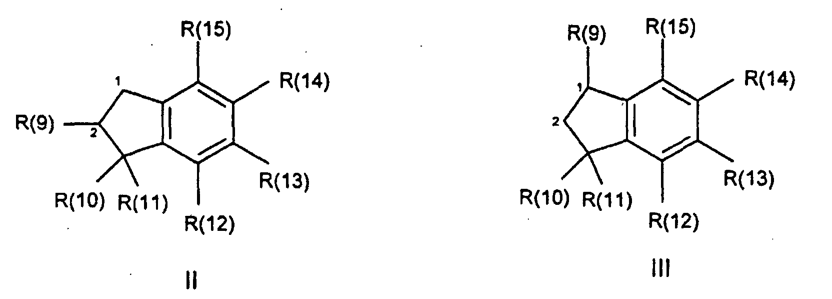 and in which:
R(1) and R(2),
independently of one another, are R(20)-CᵣH₂ᵣ, where a CH₂ group of the group CᵣH₂ᵣ may be replaced by -O-, -CH=CH-, -C≡C-, -CO-, -CO-O-, -O-CO-, -S-, -SO-, -SO₂-, -NR(21)- or -CONR(21);
R(21) is hydrogen or alkyl having 1, 2 or 3 carbon atoms;
R(20) is H, CH₃, CH₂F, CHF₂, CF₃, C₂F₅, C₃F₇, cycloalkyl having 3, 4, 5, 6, 7 or 8 carbon atoms, NR(22)R(23), -CONR(22)R(23), -OR(24), -COOR(24), phenyl or an N-containing heterocycle having 1, 2, 3, 4, 5, 6, 7, 8 or 9 carbon atoms,
where phenyl and the N-containing heterocycle are unsubstituted or substituted by 1 or 2 substituents, selected from the group consisting of F, Cl, Br, I, CF₃, NO₂, CN, NH₂, OH, methyl, ethyl, hydroxymethyl, hydroxyethyl, methoxy, dimethylamino, sulfamoyl, methylsulfonyl and methylsulfonylamino;
R(22) and R(23),
independently of one another, are hydrogen or alkyl having 1, 2, 3 or 4 carbon atoms;
or
R(22) and R(23)
together form a chain of 4 or 5 methylene groups, of which one CH₂ group can be replaced by -O-, -S-, -NH-, -N(methyl)- or -N(benzyl)-;
R(24) is hydrogen or alkyl having 1, 2 or 3 carbon atoms;
r is zero, 1, 2, 3, 4, 5, 6, 7 or 8;
or
R(1) and R(2)
together form a chain of 4 or 5 methylene groups, of which one CH₂ group can be replaced by -O-, -S-, -NH-, -N(methyl)- or -N(benzyl)-;
R(3), R(4), R(5) and R(6),
independently of one another, are hydrogen, F, Cl, Br, I, alkyl having 1, 2, 3, 4 or 5 carbon atoms, cycloalkyl having 3, 4, 5, 6, 7 or 8 carbon atoms, CN, CF₃, NO₂, OR(25) or NR(26)R(27);
R(25) is hydrogen, alkyl having 1, 2, 3 or 4 carbon atoms, a fluorinated alkyl radical of the formula -CₓH₂ₓCF_{y}H_{3-y} or phenyl, which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
x is 0, 1, 2 or 3;
y is 1, 2 or 3;
R(26) and R(27),
independently of one another, are hydrogen or alkyl having 1, 2, 3 or 4 carbon atoms;
or
R(26) and R(27)
together form a chain of 4 or 5 methylene groups, of which one CH₂ group can be replaced by -O-, -S-, -NH-, -N(methyl)- or -N(benzyl)- ;
R(7) is hydrogen or alkyl having 1, 2, 3 or 4 carbon atoms;
R(9) is hydrogen, OR(28) or OCOR(28);
R(28) is hydrogen or alkyl having 1, 2, 3 or 4 carbon atoms;
R(10) and R(11),
independently of one another, are hydrogen or alkyl having 1, 2, 3 or 4 carbon atoms;
R(12), R(13), R(14) and R(15), independently of one another, are hydrogen, F, Cl, Br, I, alkyl having 1, 2, 3, 4 or 5 carbon atoms, cycloalkyl having 3, 4, 5, 6, 7 or 8 carbon atoms, -CN, -CF₃, -C₂F₅, -C₃F₇, -N₃, -NO₂, -Y-CₛH₂ₛ-R(29), phenyl, thienyl, furyl or an N-containing heterocycle having 1, 2, 3, 4, 5, 6, 7, 8 or 9 carbon atoms,
where phenyl, thienyl, furyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
Y is -O-, -CO-, -CO-O-, -O-CO-, -S-, -SO-, -SO₂-, -O-SO₂-, -SO₂NR(30)-, -CONR(30)- or -NR(30)CO-, where the link to the backbone is in each case via the atom on the left; R(30) is hydrogen or alkyl having 1, 2 or 3 carbon atoms;
s is zero, 1, 2, 3, 4, 5 or 6;
R(29) is hydrogen, methyl, CF₃, C₂F₅, C₃F₇, cycloalkyl having 3, 4, 5, 6, 7 or 8 carbon atoms, -OR(31), -COOR(31), -NR(32)R(33), -CONR(32)R(33), phenyl or an N-containing heterocycle having 1, 2, 3, 4, 5, 6, 7, 8 or 9 carbon atoms,
where phenyl and the N-containing heterocycle are unsubstituted or substituted by 1 or 2 substituents, selected from the group consisting of F, Cl, Br, I, CF₃, NO₂, CN, NH₂, OH, methyl, ethyl, methoxy, dimethylamino, sulfamoyl, methylsulfonyl and. methylsulfonylamino;
R(31) is hydrogen or alkyl having 1, 2 or 3 carbon atoms;
R(32) and R(33), independently of one another, are hydrogen or alkyl having 1, 2, 3 or 4 carbon atoms;
or
R(32) and R(33)
together form a chain of 4 or 5 methylene groups, of which one CH₂ group can be replaced by -O-, -S-, -NH-, -N(CH₃)- or -N(benzyl)-;
and its physiologically tolerated salts.

2. A compound of the formula I as claimed in claim 1, but where at least one of the radicals R(1) and R(2) is other than hydrogen.

3. A compound of the formula I as claimed in claim 1 or 2, in which R(8) is either a 1-indanyl radical of the formula II or a 2-indanyl radical of the formula III and in which:
R(1) is hydrogen;
R(2) is R(20)-CᵣH₂ᵣ,
where one CH₂ group of the group CᵣH₂ᵣ may be replaced by -O-, -CH=CH-, -C≡C-, -CO-, -CO-O-, -O-CO-, -S-, -SO-, -SO₂-, -NR(21)- or-CONR(21); R(21) is hydrogen or alkyl having 1, 2 or 3 carbon atoms;
R(20) is CH₃, CH₂F, CHF₂, CF₃, C₂F₅, C₃F₇, cycloalkyl having 3, 4, 5, 6, 7 or 8 carbon atoms, NR(22)R(23), -CONR(22)R(23), -OR(24), -COOR(24), phenyl or an N-containing heterocycle having 1, 2, 3, 4, 5, 6, 7, 8 or 9 carbon atoms,
where phenyl and the N-containing heterocycle are unsubstituted or substituted by 1 or 2 substituents selected from the group consisting of F, Cl, Br, I, CF₃, NO₂, CN, NH₂, OH, methyl, ethyl, hydroxymethyl, hydroxyethyl, methoxy, dimethylamino, sulfamoyl, methylsulfonyl and methylsulfonylamino;
R(22) and R(23),
independently of one another, are hydrogen or alkyl having 1, 2, 3 or 4 carbon atoms;
or
R(22) and R(23) together form a chain of 4 or 5 methylene groups, of which one CH₂ group can be replaced by -O-, -S-, -NH-, -N(methyl)- or -N(benzyl)-;
R(24) is hydrogen or alkyl having 1, 2 or 3 carbon atoms;
r is zero, 1, 2, 3, 4, 5, 6, 7 or 8;
R(3), R(4), R(5) and R(6), independently of one another, are hydrogen, F, Cl, Br, I, alkyl having 1, 2, 3, 4 or 5 carbon atoms, cycloalkyl having 3, 4, 5, 6, 7 or 8 carbon atoms, CN, CF₃, NO₂, OR(25) or NR(26)R(27);
R(25) is hydrogen, alkyl having 1, 2, 3 or 4 carbon atoms, a fluorinated alkyl radical of the formula -CₓH₂ₓCF_{y}H_{3-y} or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
x is 0, 1, 2 or 3;
y is 1, 2 or 3;
R(26) and R(27), independently of one another, are hydrogen or alkyl having 1, 2, 3 or 4 carbon atoms;
or
R(26) and R(27)
together form a chain of 4 or 5 methylene groups, of which one CH₂ group can be replaced by -O-, -S-, -NH-, -N(methyl)- or -N(benzyl)-;
R(7) is hydrogen or alkyl having 1, 2, 3 or 4 carbon atoms;
R(9) is hydrogen, OR(28) or OCOR(28);
R(28) is hydrogen or alkyl having 1, 2, 3 or 4 carbon atoms;
R(10) and R(11),
independently of one another, are hydrogen or alkyl having 1, 2, 3 or 4 carbon atoms;
R(12), R(13), R(14) and R(15),
independently of one another, are hydrogen, F, Cl, Br, I, alkyl having 1, 2, 3, 4 or 5 carbon atoms, cycloalkyl having 3, 4, 5, 6, 7 or 8 carbon atoms, -CN, -CF₃, -C₂F₅, -C₃F₇, -N₃, -NO₂, -Y-CₛH₂ₛ-R(29), phenyl, thienyl, furyl or an N-containing heterocycle having 1, 2, 3, 4, 5, 6, 7, 8 or 9 carbon atoms,
where phenyl, thienyl, furyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
Y is -O-, -CO-, -CO-O-, -O-CO-, -S-, -SO-, -SO₂-, -O-SO₂-, -SO₂NR(30)-, -CONR(30)- or -NR(30)CO-, where the link to the backbone is in each case via the atom on the left; R(30) is hydrogen or alkyl having 1, 2 or 3 carbon atoms;
s is zero, 1, 2, 3, 4, 5 or 6;
R(29) is hydrogen, methyl, CF₃, C₂F₅, C₃F₇, cycloalkyl having 3, 4, 5, 6, 7 or 8 carbon atoms, -OR(31), -COOR(31), -NR(32)R(33), -CONR(32)R(33), phenyl or an N-containing heterocycle having 1, 2, 3, 4, 5, 6, 7, 8 or 9 carbon atoms,
where phenyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(31) is hydrogen or alkyl having 1, 2 or 3 carbon atoms;
R(32) and R(33), independently of one another, are hydrogen or alkyl having 1, 2, 3 or 4 carbon atoms;
or
R(32) and R(33)
together form a chain of 4 or 5 methylene groups, of which one CH₂ group can be replaced by -O-, -S-, -NH-, -N(CH₃)- or -N(benzyl)-;
and its physiologically tolerated salts.

4. A compound of the formula I as claimed in one or more of claims 1 to 3, in which R(8) is a 1-indanyl radical of the formula II, i.e. compounds of the formula la 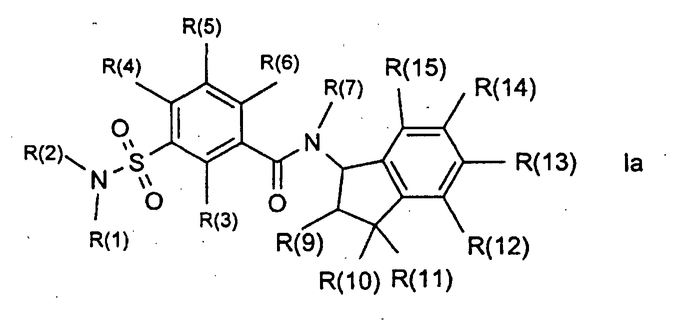 in which:
R(1) is hydrogen;
R(2) is R(20)-CYᵣH₂ᵣ,
where one CH₂ group of the group CᵣH₂ᵣ may be replaced by -O-, -CH=CH-, -C≡C-, -CO-, -CO-O-, -O-CO-, -S-, -SO-, -SO₂-, -NR(21)- or-CONR(21); R(21) is hydrogen or alkyl having 1, 2 or 3 carbon atoms;
R(20) is CH₃, CH₂F, CHF₂, CF₃, C₂F₅, C₃F₇, cycloalkyl having 3, 4, 5, 6, 7 or 8 carbon atoms, NR(22)R(23), -CONR(22)R(23), -OR(24), -COOR(24), phenyl or an N-containing heterocycle having 1, 2, 3, 4, 5, 6, 7, 8 or 9 carbon atoms,
where phenyl and the N-containing heterocycle are unsubstituted or substituted by 1 or 2 substituents selected from the group consisting of F, Cl, Br, I, CF₃, NO₂, CN, NH₂, OH, methyl, ethyl, hydroxymethyl, hydroxyethyl, methoxy, dimethylamino, sulfamoyl, methylsulfonyl and methylsulfonylamino;
R(22) and R(23), independently of one another, are hydrogen or alkyl having 1, 2, 3 or 4 carbon atoms;
or
R(22) and R(23) together form a chain of 4 or 5 methylene groups, of which one CH₂ group can be replaced by -O-, -S-, -NH-, -N(methyl)- or -N(benzyl)-;
R(24) is hydrogen or alkyl having 1, 2 or 3 carbon atoms;
r is zero, 1, 2, 3, 4, or 5;
R(3), R(4), R(5) and R(6), independently of one another, are hydrogen, F, Cl, Br, I, alkyl having 1, 2, 3, 4 or 5 carbon atoms, cycloalkyl having 3, 4, 5, 6, 7 or 8 carbon atoms, CN, CF₃, NO₂ or OR(25);
R(25) is hydrogen, alkyl having 1, 2, 3 or 4 carbon atoms, a fluorinated alkyl radical of the formula -CₓH₂ₓCF_{y}H_{3-y} or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
x is 0, 1, 2 or 3;
y is 1, 2 or 3;
R(7) is hydrogen or alkyl having 1, 2, 3 or 4 carbon atoms;
R(9) is hydrogen, OR(28) or OCOR(28);
R(28) is hydrogen or alkyl having 1, 2, 3 or 4 carbon atoms;
R(10) and R(11), independently of one another, are hydrogen or alkyl having 1, 2, 3 or 4 carbon atoms;
R(12), R(13), R(14) and R(15), independently of one another, are hydrogen, F, Cl, Br, I, alkyl having 1, 2, 3, 4 or 5 carbon atoms, cycloalkyl having 3, 4, 5, 6, 7 or 8 carbon atoms, -CN, -CF₃, -C₂F₅, -C₃F₇, -NO₂, -Y-CₛH₂ₛ-R(29), phenyl, thienyl, furyl or an N-containing heterocycle having 1, 2, 3, 4, 5, 6, 7, 8 or 9 carbon atoms,
where phenyl, thienyl, furyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
Y is -O-, -CO-, -CO-O-, -O-CO-, -S-, -SO-, -SO₂-, -O-SO₂-, -SO₂NR(30)-, -CONR(30)- or -NR(30)CO-, where the link to the backbone is in each case via the atom on the left; R(30) is hydrogen or alkyl having 1, 2 or 3 carbon atoms;
s is zero, 1, 2, 3, 4, 5 or 6;
R(29) is hydrogen, methyl, CF₃, C₂F₅, C₃F₇, cycloalkyl having 3, 4, 5, 6, 7 or 8 carbon atoms, -OR(31), -COOR(31), -NR(32)R(33), -CONR(32)R(33), phenyl or an N-containing heterocycle having 1, 2, 3, 4, 5, 6, 7, 8 or 9 carbon atoms,
where phenyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(31) is hydrogen or alkyl having 1, 2 or 3 carbon atoms;
R(32) and R(33), independently of one another, are hydrogen or alkyl having 1, 2, 3 or 4 carbon atoms;
or
R(32) and R(33) together form a chain of 4 or 5 methylene groups, of which one CH₂ group can be replaced by -O-, -S-, -NH-, -N(CH₃)- or -N(benzyl)-; .
and its physiologically tolerated salts

5. A compound of the formula la as claimed in one or more of claims 1 to 4, in which:
R(1) is hydrogen;
R(2) is R(20)-CᵣH₂ᵣ;
R(20) is CH₃, CH₂F, CHF₂, CF₃, cycloalkyl having 3, 4, 5, 6, 7 or 8 carbon atoms, -CONR(22)R(23), -OR(24), -COOR(24) or phenyl,
which is unsubstituted or substituted by 1 or 2 substituents selected from the group consisting of F, Cl, Br, CF₃, NO₂, CN, OH, methyl, ethyl, hydroxymethyl, hydroxyethyl, methoxy, dimethylamino, sulfamoyl, methylsulfonyl and methylsulfonylamino;
R(22) and R(23), independently of one another, are hydrogen or alkyl having 1, 2, 3 or 4 carbon atoms;
or
R(22) and R(23)
together form a chain of 4 or 5 methylene groups, of which one CH₂ group can be replaced by -O-, -S-, -NH-, -N(methyl)- or -N(benzyl)-;
R(24) is hydrogen or alkyl having 1, 2 or 3 carbon atoms;
r is zero, 1, 2, 3, 4, or 5;
R(3), R(4), R(5) and R(6), independently of one another, are hydrogen, F, Cl, Br, I, alkyl having 1, 2, 3, 4 or 5 carbon atoms, cycloalkyl having 3, 4, 5, 6, 7 or 8 carbon atoms, CN, CF₃, NO₂ or OR(25);
R(25) is hydrogen, alkyl having 1, 2, 3 or 4 carbon atoms, a fluorinated alkyl radical of the formula -CₓH₂ₓCF_{y}H_{3-y} or phenyl,
which is unsubstituted or substituted by 1 or 2 substituents selected from the group consisting of F, Cl, Br, CF₃, NO₂, CN, OH, methyl, ethyl, methoxy, dimethylamino, sulfamoyl, methylsulfonyl and methylsulfonylamino;
x is 0, 1, 2 or 3;
y is 1, 2 or 3;
R(7) is hydrogen or alkyl having 1, 2, 3 or 4 carbon atoms;
R(9) is hydrogen or OR(28);
R(28) is hydrogen or alkyl having 1, 2, 3 or 4 carbon atoms;
R(10) and R(11),
independently of one another, are hydrogen or alkyl having 1, 2, 3 or 4 carbon atoms;
R(12), R(13), R(14) and R(15), independently of one another, are hydrogen, F, Cl, Br, I, alkyl having 1, 2, 3, 4 or 5 carbon atoms, cycloalkyl having 3, 4, 5, 6, 7 or 8 carbon atoms, CN, CF₃, -NO₂ or -Y-CₛH₂ₛ-R(29);
Y is -O-, -CO-, -CO-O-, -O-CO-, -S-, -SO-, -SO₂-, -O-SO₂-, -SO₂NR(30)-, -CONR(30)- or -NR(30)CO-, where the link to the backbone is in each case via the atom on the left; R(30) is hydrogen or alkyl having 1, 2 or 3 carbon atoms;
s is zero, 1, 2, 3, 4 or 5;
R(29) is hydrogen, methyl, CF₃, -OR(31), -COOR(31), -NR(32)R(33), -CONR(32)R(33) or phenyl,
which is unsubstituted or substituted by 1 or 2 substituents selected from the. group consisting of F, Cl, Br, CF₃, NO₂, CN, OH, methyl, ethyl, methoxy, dimethylamino, sulfamoyl, methylsulfonyl and methylsulfonylamino;
R(31) is hydrogen or alkyl having 1, 2 or 3 carbon atoms;
R(32) and R(33),
independently of one another, are hydrogen or alkyl having 1, 2, 3 or 4 carbon atoms;
or
R(32) and R(33)
together form a chain of 4 or 5 methylene groups, of which one CH₂ group can be replaced by -O-, -S-, -NH-, -N(CH₃)- or -N(benzyl)-;
and its physiologically tolerated salts.

6. A process for the preparation of compounds of the formula I as claimed in one or more of claims 1 to 5, which comprises reacting a carboxylic acid of the formula IV 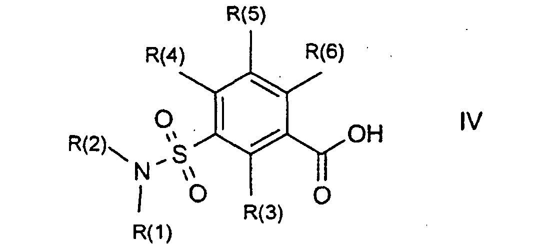 in which R(1), R(2), R(3), R(4), R(5) and R(6) have the meanings stated in claims 1 to 5, with an amine of the formula V a or V b 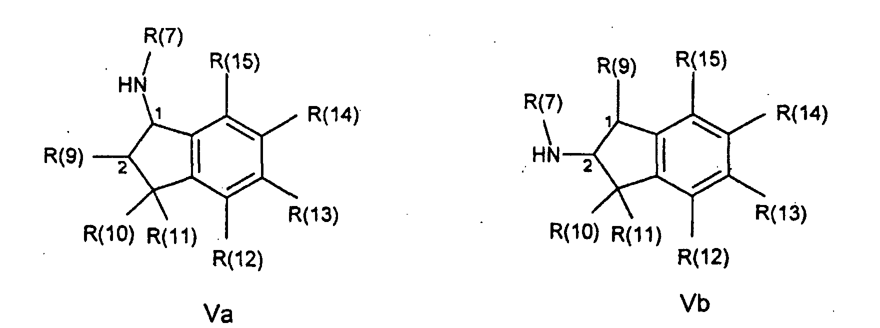 in which R(7), R(9), R(10), R(11), R(12), R(13), R(14) and R(15) have the meanings stated in claims 1 to 6, in an amidation reaction.

7. A compound of the formula I as claimed in one or more of claims 1 to 5 and its physiologically tolerated salts for use as medicaments.

8. A pharmaceutical formulation containing an effective amount of at least one compound of the formula I as claimed in one or more of claims 1 to 5 and/or one physiologically tolerated salt thereof as an active substance, together with pharmaceutically acceptable carriers and additives and, if required, also one or more other pharmacological active substances.

9. The use of a compound of the formula 1 as claimed in one or more of claims 1 to 5 and/or a physiologically tolerated salt thereof for the preparation of a medicament having a K ⁺-channel-blocking effect for the therapy and prophylaxis of K⁺-channel mediated diseases.

10. The use of a compound of the formula I as claimed in one or more of claims 1 to 5 and/or a physiologically tolerated salt thereof for the preparation of a medicament for the therapy or prophylaxis of cardiac arrhythmias which can be eliminated by lengthening the action potential.

11. The use of a compound of the formula I as claimed in one or more of claims 1 to 5 and/or a physiologically tolerated salt thereof for the preparation of a medicament for the therapy or prophylaxis of re-entry arrhythmias.

12. The use of a compound of the formula I as claimed in one or more of claims 1 to 5 and/or a physiologically tolerated salt thereof for the preparation of a medicament for the therapy or prophylaxis of supraventricular arrhythmias.

13. The use of a compound of the formula I as claimed in one or more of claims 1 to 5 and/or a physiologically tolerated salt thereof for the preparation of a medicament for the therapy or prophylaxis of atrial fibrillation or atrial flutter.

14. A pharmaceutical formulation containing an effective amount of at least one compound of the formula I as claimed in one or more of claims 1 to 5 and/or of one physiologically tolerated salt thereof and of a beta-blocker as active substances, together with pharmaceutically acceptable carriers and additives.

15. A pharmaceutical formulation containing an effective amount of at least one compound of the formula I as claimed in one or more of claims 1 to 5 and/or of a physiologically tolerated salt thereof and of an IKₛ-channel blocker as active substances, together with pharmaceutically acceptable carriers and additives.

## Revendications

1. Composés de la formule I : 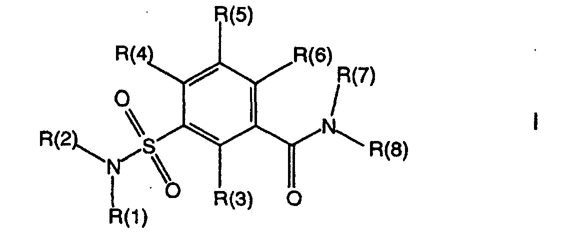 dans laquelle R(8) représente un reste 1-indanyle de la formule II ou un reste 2-indanyle de la formule III : 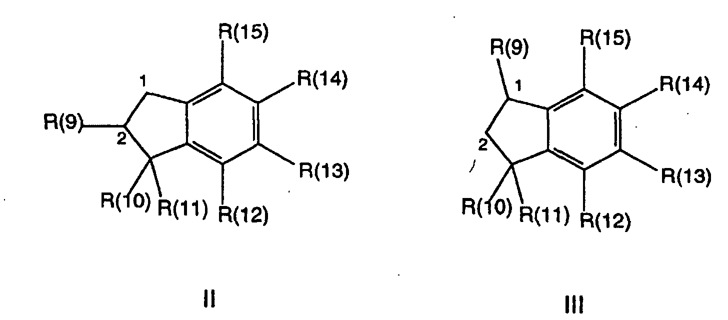 et où :
R(1) et R(2) signifient indépendamment l'un de l'autre, R(20)-CᵣH₂ᵣ :
où un des radicaux CH₂ du radical CᵣH₂ᵣ peut être remplacé par -O-, -CH=CH-, -C≡C-, -CO-, -CO-O--O-CO-, -S-, -SO-, -SO₂-, -NR(21)- ou -CONR(21)- ;
R(21) représente hydrogène ou alkyle ayant 1, 2 ou 3 atomes C ;
R(20) représente H, CH₃, CH₂F, CHF₂, CF₃, C₂F₅, C₃F₇, cycloalkyle ayant 3, 4, 5, 6, 7 ou 8 atomes C, NR(22)R(23), -CONR(22)R(23), -OR(24), -COOR(24), phényle ou un hétérocycle azoté ayant 1, 2, 3, 4, 5, 6, 7, 8 ou 9 atomes C,
où le phényle et l'hétérocycle azoté sont non substitués ou substitués avec 1 ou 2 substituants, choisis parmi le groupe consistant en F, Cl, Br, I, CF₃, NO₂, CN, NH₂, OH, méthyle, éthyle, hydroxyméthyle, hydroxyéthyle, méthoxy, diméthylamino, sulfamoyle, méthylsulfonyle et méthylsulfonylamino ;
R(22) et R(23) représentent indépendamment l'un de l'autre, hydrogène ou alkyle ayant 1, 2, 3 ou 4 atomes C ;
ou
R(22) et R(23) représentent ensemble, une chaîne de 4 ou 5 radicaux méthylène, dont un radical CH₂ peut être remplacé par -O-, -S-, -NH-, -N(méthyle)- ou -N(benzyle)- ;
R(24) représente hydrogène ou alkyle ayant 1, 2 ou 3 atomes C ;
r est zéro, 1, 2, 3, 4, 5, 6, 7 ou 8 ;
ou R(1) et R(2) représentent ensemble, une chaîne de 4 ou 5 radicaux méthylène, dont un radical CH₂ peut être remplacé par -O-, -S-, -NH-, -N(méthyle)- ou -N(benzyle)- ;
R(3), R(4), R(5) et R(6) représentent indépendamment l'un de l'autre, hydrogène, F, Cl, Br, I, alkyle ayant 1, 2, 3, 4 ou 5 atomes C, cycloalkyle ayant 3, 4, 5, 6, 7 ou 8 atomes C, CN, CF₃, NO₂, OR(25) ou NR(26)R(27) ;
R(25) représente hydrogène ou alkyle ayant 1, 2, 3 ou 4 atomes C, un reste alkyle fluoré de la formule -CₓH₂ₓCF_{y}H_{3-y} ou phényle, qui est non substitué ou substitué par 1 ou 2 substituants, choisis parmi le groupe consistant en F, Cl, Br, I, CF₃, NO₂, CN, NH₂, OH, méthyle, éthyle, méthoxy, diméthylamino, sulfamoyle, méthylsulfonyle et méthylsulfonylamine ;
x est 0, 1, 2 ou 3 ;
y est 1, 2 ou 3 ;
R(26) et R(27) représentent indépendamment l'un de l'autre, hydrogène ou alkyle ayant 1, 2, 3 ou 4 atomes C ;
ou R(26) et R(27) représentent ensemble, une chaîne de 4 ou 5 radicaux méthylène, dont un radical CH₂ peut être remplacé par -O-, -S-, -NH-, -N(méthyle)- ou -N(benzyle)- ;
R(7) représente hydrogène ou alkyle ayant 1, 2, 3 ou 4 atomes C ;
R(9) représente hydrogène, OR(28) ou OCOR(28) ;
R(28) représente hydrogène ou alkyle ayant 1, 2, 3 ou 4 atomes C ;
R(10) et R(11) représentent indépendamment l'un de l'autre, hydrogène ou alkyle ayant 1, 2, 3 ou 4 atomes C ;
R(12), R(13), R(14) et R(15) représentent indépendamment l'un de l'autre, hydrogène, F, Cl, Br, I, alkyle ayant 1, 2, 3, 4 ou 5 atomes C, cycloalkyle ayant 3, 4, 5, 6, 7 ou 8 atomes C, CN, CF₃, C₂F₅, C₃F₇, N₃, NO₂, Y-CₛH₂ₛ-R(29), phényle, thiényle, furyle ou un hétérocycle azoté ayant 1, 2, 3, 4, 5, 6, 7, 8 ou 9 atomes C,
où les phényle, thiényle, furyle et l'hétérocycle azoté sont non substitués ou substitués avec 1 ou 2 substituants, choisis parmi le groupe consistant en F, Cl, Br, I, CF₃, NO₂, CN, NH₂, OH, méthyle, éthyle, méthoxy, diméthylamino, sulfamoyle, méthylsulfonyle et méthylsulfonylamino ;
Y représente -O-, -CO-, -CO-O- -O-CO-, -S-, -SO-, -SO₂-, -O-SO₂-, -SO₂NR(30)-, -CONR(30)- ou -NR(30)CO-, où la liaison avec le squelette de base est chaque fois réalisée par l'atome situé à gauche ;
R(30) représente hydrogène ou alkyle ayant 1, 2 ou 3 atomes C ;
s est zéro, 1, 2, 3, 4, 5 ou 6 ;
R(29) représente hydrogène, méthyle, CF₃, C₂F₅, C₃F₇, cycloalkyle ayant 3, 4, 5, 6, 7 ou 8 atomes C, OR(31), COOR(31), NR(32)R(33), CONR(32)R(33), phényle ou un hétérocycle azoté ayant 1, 2, 3, 4, 5, 6, 7, 8 ou 9 atomes C,
où le phényle et l'hétérocycle azoté sont non substitués ou substitués avec 1 ou 2 substituants, choisis parmi le groupe consistant en F, Cl, Br, I, CF₃, NO₂, CN, NH₂, OH, méthyle, éthyle, méthoxy, diméthylamino, sulfamoyle, méthylsulfonyle et méthylsulfonylamino ;
R(31) représente hydrogène ou alkyle ayant 1, 2 ou 3 atomes C ;
R(32) et R(33) représentent indépendamment l'un de l'autre, hydrogèhe ou alkyle ayant 1, 2, 3 ou 4 atomes C, ou
R(32) et R(33) représentent ensemble, une chaîne de 4 ou 5 radicaux méthylène, dont un radical CH₂ peut être remplacé par -O-, -S-, -NH-, -N(CH₃)- ou -N(benzyle)- ;
ainsi que leurs sels physiologiquement compatibles.

2. Composés de la formule I selon la revendication 1, où cependant au moins un des restes R(1) ou R(2) a une autre signification que hydrogène.

3. Composés de la formule I selon les revendications 1 ou 2, dans lesquels R(8) représente un reste 1-indanyle de la formule II ou un reste 2-indanyle de la formule III, et où :
R(1) représente hydrogène ;
R(2) représente R(20)-CᵣH₂ᵣ,
où un des radicaux CH₂ du radical CᵣH₂ᵣ peut être remplacé par -O-, -CH=CH-, -C≡C-, -CO-, -CO-O--O-CO-, -S-, -SO-, -SO₂-, -NR(21)- ou -CONR(21)- ;
R(21) représente hydrogène ou alkyle ayant 1, 2 ou 3 atomes C ;
R(20) représente CH₃, CH₂F, CHF₂, CF₃, C₂F₅, C₃F₇, cycloalkyle ayant 3, 4, 5, 6, 7 ou 8 atomes C, NR(22)R(23), CONR(22)R(23), OR(24), COOR(24), phényle ou un hétérocycle azoté ayant 1, 2, 3, 4, 5, 6, 7, 8 ou 9 atomes C,
où le phényle et l'hétérocycle azoté sont non substitués ou substitués avec 1 ou 2 substituants, choisis parmi le groupe consistant en F, Cl, Br, I, CF₃, NO₂, CN, NH₂, OH, méthyle, éthyle, hydroxyméthyle, hydroxyéthyle, méthoxy, diméthylamino, sulfamoyle, méthylsulfonyle et méthylsulfonylamino ;
R(22) et R(23) représentent indépendamment l'un de l'autre, hydrogène ou alkyle ayant 1, 2, 3 ou 4 atomes C ;
ou
R(22) et R(23) représentent ensemble, une chaîne de 4 ou 5 radicaux méthylène, dont un radical CH₂ peut être remplacé par -O-, -S-, -NH-, -N(méthyle)- ou -N(benzyle)- ;
R(24) représente hydrogène ou alkyle ayant 1, 2 ou 3 atomes C ;
r est zéro, 1, 2, 3, 4, 5, 6, 7 ou 8 ;
R(3), R(4), R(5) et R(6) représentent indépendamment l'un de l'autre, hydrogène, F, Cl, Br, I, alkyle ayant 1, 2, 3, 4 ou 5 atomes C, cycloalkyle ayant 3, 4, 5, 6, 7 ou 8 atomes C, CN, CF₃, NO₂, OR(25) ou NR(26)R(27) ;
R(25) représente hydrogène ou alkyle ayant 1, 2, 3 ou 4 atomes C, un reste alkyle fluoré de la formule -CₓH₂ₓCF_{y}H_{3-y} ou phényle, qui est non substitué ou substitué par 1 ou 2 substituants, choisis parmi le groupe consistant en F, Cl, Br, I, CF₃, NO₂, CN, NH₂, OH, méthyle, éthyle, méthoxy, diméthylamino, sulfamoyle, méthylsulfonyle et méthylsulfonylamino ;
x est 0, 1, 2 ou 3 ;
y est 1, 2 ou 3 ;
R(26) et R(27) représentent indépendamment l'un de l'autre, hydrogène ou alkyle ayant 1, 2, 3 ou 4 atomes C ;
ou
R(26) et R(27) représentent ensemble, une chaîne de 4 ou 5 radicaux méthylène, dont un radical CH₂ peut être remplacé par -O-, -S-, -NH-, -N(méthyle)- ou -N(benzyle)- ;
R(7) représente hydrogène ou alkyle ayant 1, 2, 3 ou 4 atomes C ;
R(9) représente hydrogène, OR(28) ou OCOR(28) ;
R(28) représente hydrogène ou alkyle ayant 1, 2, 3 ou 4 atomes C ;
R(10) et R(11) représentent indépendamment l'un de l'autre, hydrogène ou alkyle ayant 1, 2, 3 ou 4 atomes C ;
R(12), R(13), R(14) et R(15) représentent indépendamment l'un de l'autre, hydrogène, F, Cl, Br, I, alkyle ayant 1, 2, 3, 4 ou 5 atomes C, cycloalkyle ayant 3, 4, 5, 6, 7 ou 8 atomes C, CN, CF₃, C₂F₅, C₃F₇, N₃, NO₂, Y-CₛH₂ₛ-R(29), phényle, thiényle, furyle ou un hétérocycle azoté ayant 1, 2, 3, 4, 5, 6, 7, 8 ou 9 atomes C,
où les phényle, thiényle, furyle et l'hétérocycle azoté sont non substitués ou substitués avec 1 ou 2 substituants, choisis parmi le groupe consistant en F, Cl, Br, I, CF₃, NO₂, CN, NH₂, OH, méthyle, éthyle, méthoxy, diméthylamino, sulfamoyle, méthylsulfonyle et méthylsulfonylamino ;
Y représente -O-, -CO-, -CO-O- -O-CO-, -S-, -SO-, -SO₂-, -O-SO₂-, -SO₂NR(30)-,-CONR(30)- ou -NR(30)CO-, où la liaison avec le squelette de base est chaque fois réalisée par l'atome situé à gauche ;
R(30) représente hydrogène ou alkyle ayant 1, 2 ou 3 atomes C ; s est zéro, 1, 2, 3, 4, 5 ou 6 ;
R(29) représente hydrogène, méthyle, CF₃, C₂F₅, C₃F₇, cycloalkyle ayant 3, 4, 5, 6, 7 ou 8 atomes C, OR(31), COOR(31), NR(32)R(33), CONR(32)R(33), phényle ou un hétérocycle azoté ayant 1, 2, 3, 4, 5, 6, 7, 8 ou 9 atomes C,
où le phényle et l'hétérocycle azoté sont non substitués ou substitués avec 1 ou 2 substituants, choisis parmi le groupe consistant en F, Cl, Br, I, CF₃, NO₂, CN, NH₂, OH, méthyle, éthyle, méthoxy, diméthylamino, sulfamoyle, méthylsulfonyle et méthylsulfonylamino ;
R(31) représente hydrogène ou alkyle ayant 1, 2 ou 3 atomes C ;
R(32) et R(33) représentent indépendamment l'un de l'autre, hydrogène ou alkyle ayant 1, 2, 3 ou 4 atomes C, ou
R(32) et R(33) représentent ensemble, une chaîne de 4 ou 5 radicaux méthylène, dont un radical CH₂ peut être remplacé par -O-, -S-, -NH-, -N(CH₃)- ou -N(benzyle)- ;
ainsi que leurs sels physiologiquement compatibles.

4. Composés de la formule I selon l'une ou plusieurs des revendications 1 à 3, dans lesquels R(8) représente un reste 1-indanyle de la formule II, à savoir les composés de la formule Ia : 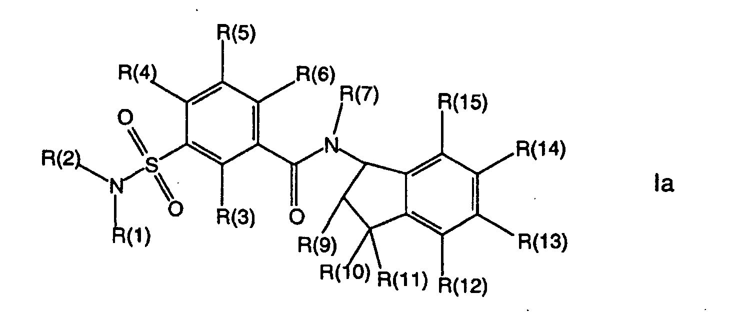 où :
R(1) représente hydrogène ;
R(2) représente R(20)-CᵣH₂ᵣ,
où un des radicaux CH₂ du radical CᵣH₂ᵣ peut être remplacé par -O-, -CH=CH-, -C≡C-, -CO-, -CO-O--O-CO-, -S-, -SO-, -SO₂-, -NR(21)- ou -CONR(21)- ;
R(21) représente hydrogène ou alkyle ayant 1, 2 ou 3 atomes C ;
R(20) représente CH₃, CH₂F, CHF₂, CF₃, C₂F₅, C₃F₇, cycloalkyle ayant 3, 4, 5, 6, 7 ou 8 atomes C, NR(22)R(23), CONR(22)R(23), OR(24), COOR(24), phényle ou un hétérocycle azoté ayant 1, 2, 3, 4, 5, 6, 7, 8 ou 9 atomes C,
où le phényle et l'hétérocycle azoté sont non substitués ou substitués avec 1 ou 2 substituants, choisis parmi le groupe consistant en F, Cl, Br, I, CF₃, NO₂, CN, NH₂, OH, méthyle, éthyle, hydroxyméthyle, hydroxyéthyle, méthoxy, diméthylamino, sulfamoyle, méthylsulfonyle et méthylsulfonylamino ;
R(22) et R(23) représentent indépendamment l'un de l'autre, hydrogène ou alkyle ayant 1, 2, 3 ou 4 atomes C ;
ou
R(22) et R(23) représentent ensemble, une chaîne de 4 ou 5 radicaux méthylène, dont un radical CH₂ peut être remplacé par -O-, -S-, -NH-, -N(méthyle)- ou -N(benzyle)- ;
R(24) représente hydrogène ou alkyle ayant 1, 2 ou 3 atomes C ;
r est zéro, 1, 2, 3, 4 ou 5 ;
R(3), R(4), R(5) et R(6) représentent indépendamment l'un de l'autre, hydrogène, F, Cl, Br, I, alkyle ayant 1, 2, 3, 4 ou 5 atomes C, cycloalkyle ayant 3, 4, 5, 6, 7 ou 8 atomes C, CN, CF₃, NO₂ ou OR(25) ;
R(25) représente hydrogène ou alkyle ayant 1, 2, 3 ou 4 atomes C, un reste alkyle fluoré de la formule -CₓH₂ₓCF_{y}H_{3-y} ou phényle, qui est non substitué ou substitué par 1 ou 2 substituants, choisis parmi le groupe consistant en F, Cl, Br, I, CF₃, NO₂, CN, NH₂, OH, méthyle, éthyle, méthoxy, diméthylamino, sulfamoyle, méthylsulfonyle et méthylsulfonylamino ;
x est 0, 1, 2 ou 3 ;
y est 1, 2 ou 3 ;
R(7) représente hydrogène ou alkyle ayant 1, 2, 3 ou 4 atomes C ;
R(9) représente hydrogène, OR(28) ou OCOR(28) ;
R(28) représente hydrogène ou alkyle ayant 1, 2, 3 ou 4 atomes C ;
R(10) et R(11) représentent indépendamment l'un de l'autre, hydrogène ou alkyle ayant 1, 2, 3 ou 4 atomes C ;
R(12), R(13), R(14) et R(15) représentent indépendamment l'un de l'autre, hydrogène, F, Cl, Br, I, alkyle ayant 1, 2, 3, 4 ou 5 atomes C, cycloalkyle ayant 3, 4, 5, 6, 7 ou 8 atomes C, CN, CF₃, C₂F₅, C₃F₇, NO₂, Y-CₛH₂ₛ-R(29), phényle, thiényle, furyle ou un hétérocycle azoté ayant 1, 2, 3, 4, 5, 6, 7, 8 ou 9 atomes C, où les phényle, thiényle, furyle et l'hétérocycle azoté sont non substitués ou substitués avec 1 ou 2 substituants, choisis parmi le groupe consistant en F, Cl, Br, I, CF₃, NO₂, CN, NH₂, OH, méthyle, éthyle, méthoxy, diméthylamino, sulfamoyle, méthylsulfonyle et méthylsulfonylamino ; Y représente -O-, -CO-, -CO-O- -O-CO-, -S-, -SO-, -SO₂-, -O-SO₂-, -SO₂NR(30)-,-CONR(30)- ou -NR(30)CO-, où la liaison avec le squelette de base est chaque fois réalisée par l'atome situé à gauche ;
R(30) représente hydrogène ou alkyle ayant 1, 2 ou 3 atomes C ;
s est zéro, 1, 2, 3, 4, 5 ou 6 ;
R(29) représente hydrogène, méthyle, CF₃, C₂F₅, C₃F₇, cycloalkyle ayant 3, 4, 5, 6, 7 ou 8 atomes C, OR(31), COOR(31), NR(32)R(33), CONR(32)R(33), phényle ou un hétérocycle azoté ayant 1, 2, 3, 4, 5, 6, 7, 8 ou 9 atomes C,
où le phényle et l'hétérocycle azoté sont non substitués ou substitués avec 1 ou 2 substituants, choisis parmi le groupe consistant en F, Cl, Br, I, CF₃, NO₂, CN, NH₂, OH, méthyle, éthyle, méthoxy, diméthylamino, sulfamoyle, méthylsulfonyle et méthylsulfonylamino ;
R(31) représente hydrogène ou alkyle ayant 1, 2 ou 3 atomes C ;
R(32) et R(33) représentent indépendamment l'un de l'autre, hydrogène ou alkyle ayant 1, 2, 3 ou 4 atomes C, ou
R(32) et R(33) représentent ensemble, une chaîne de 4 ou 5 radicaux méthylène, dont un radical CH₂ peut être remplacé par -O-, -S-, -NH-, -N(CH₃)- ou -N(benzyle)- ;
ainsi que leurs sels physiologiquement compatibles.

5. Composés de la formule I selon l'une ou plusieurs des revendications 1 à 4,
où :
R(1) représente hydrogène ;
R(2) représente R(20)-CᵣH₂ᵣ,
R(20) représente CH₃, CH₂F, CHF₂, CF₃, cycloalkyle ayant 3, 4, 5, 6, 7 ou 8 atomes C, CONR(22)R(23), OR(24), COOR(24), phényle qui est non substitué ou substitué avec 1 ou 2 substituants, choisis parmi le groupe consistant en F, Cl, Br, CF₃, NO₂, CN, OH, méthyle, éthyle, hydroxyméthyle, hydroxyéthyle, méthoxy, diméthylamino, sulfamoyle, méthylsulfonyle et méthylsulfonylamino ;
R(22) et R(23) représentent indépendamment l'un de l'autre, hydrogène ou alkyle ayant 1, 2, 3 ou 4 atomes C ;
ou
R(22) et R(23)
représentent ensemble, une chaîne de 4 ou 5 radicaux méthylène, dont un radical CH₂ peut être remplacé par -O-, -S-, -NH-, -N(méthyle)- ou -N(benzyle)- ;
R(24) représente hydrogène ou alkyle ayant 1, 2 ou 3 atomes C ;
r est zéro, 1, 2, 3, 4 ou 5 ; R(3), R(4), R(5) et R(6) représentent indépendamment l'un de l'autre, hydrogène, F, Cl, Br, I, alkyle ayant 1, 2, 3, 4 ou 5 atomes C, cycloalkyle ayant 3, 4, 5, 6, 7 ou 8 atomes C, CN, CF₃, NO₂ ou OR(25) ;
R(25) représente hydrogène ou alkyle ayant 1, 2, 3 ou 4 atomes C, un reste alkyle fluoré de la formule -CₓH₂ₓCF_{y}H_{3-y} ou phényle, qui est non substitué ou substitué par 1 ou 2 substituants, choisis parmi le groupe consistant en F, Cl, Br, CF₃, NO₂, CN, OH, méthyle, éthyle, méthoxy, diméthylamino, sulfamoyle, méthylsulfonyle et méthylsulfonylamino ;
x est 0, 1, 2 ou 3 ;
y est 1, 2 ou 3 ;
R(7) représente hydrogène ou alkyle ayant 1, 2, 3 ou 4 atomes C ; R(9) représente hydrogène, OR(28) ;
R(28) représente hydrogène ou alkyle ayant 1, 2, 3 ou 4 atomes C ;
R(10) et R(11) représentent indépendamment l'un de l'autre, hydrogène ou alkyle ayant 1, 2, 3 ou 4 atomes C ;
R(12), R(13), R(14) et R(15) représentent indépendamment l'un de l'autre, hydrogène, F, Cl, Br, I, alkyle ayant 1, 2, 3, 4 ou 5 atomes C, cycloalkyle ayant 3, 4, 5, 6, 7 ou 8 atomes C, CN, CF₃, NO₂ ou Y-CₛH₂ₛ-R(29), Y représente -O-, -CO-, -CO-O- -O-CO-, -S-, -SO-, -SO₂-, -O-SO₂-, -SO₂NR(30)-, -CONR(30)- ou -NR(30)CO-, où la liaison avec le squelette de base est chaque fois réalisée par l'atome situé à gauche ;
R(30) représente hydrogène ou alkyle ayant 1, 2 ou 3 atomes C ;
s est zéro, 1, 2, 3, 4 ou 5 ;
R(29) représente hydrogène, méthyle, CF₃, OR(31), COOR(31), NR(32)R(33), CONR(32)R(33) ou phényle qui est non substitué ou substitué avec 1 ou 2 substituants, choisis parmi le groupe consistant en F, Cl, Br, CF₃, NO₂, CN, OH, méthyle, éthyle, méthoxy, diméthylamino, sulfamoyle, méthylsulfonyle et méthylsulfonylamino ;
R(31) représente hydrogène ou alkyle ayant 1, 2 ou 3 atomes C ;
R(32) et R(33) représentent indépendamment l'un de l'autre, hydrogène ou alkyle ayant 1, 2, 3 ou 4 atomes C, ou
R(32) et R(33) représentent ensemble, une chaîne de 4 ou 5 radicaux méthylène, dont un radical CH₂ peut être remplacé par -O-, -S-, -NH-, -N(CH₃)- ou -N(benzyle)- ;
ainsi que leurs sels physiologiquement compatibles.

6. Procédé de préparation de composés de la formule I selon l'une ou plusieurs des revendications 1 à 5, **caractérisé en ce que** l'on fait réagir un acide carboxylique de la formule (IV) : 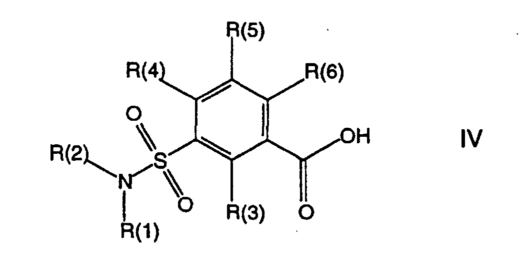 où R(1), R(2), R(3), R(4), R(5) et R(6) possèdent les significations indiquées dans les revendications 1 à 5, dans une réaction d'amidation avec une amine de la formule Va ou Vb : 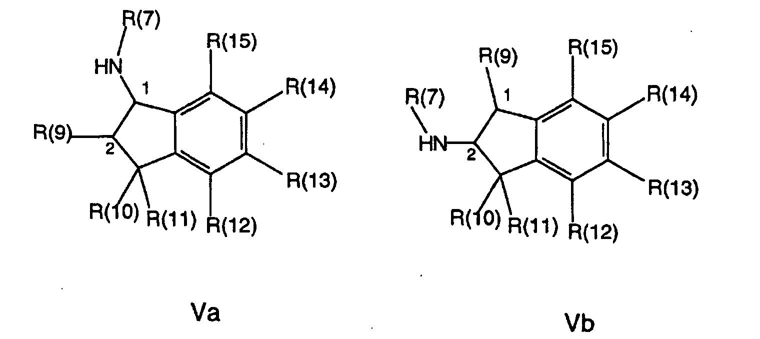 où R(7), R(9), R(10), R(11), R(12), R(13), R(14) et R(15) possèdent les significations indiquées dans les revendications 1 à 5.

7. Composés de la formule I selon une ou plusieurs des revendications 1 à 5 et leurs sels physiologiquement compatibles à utiliser comme agent pharmaceutique.

8. Composition pharmaceutique, contenant une quantité active d'au moins un composé de la formule I selon une ou plusieurs des revendications 1 à 5 et/ou un sel physiologiquement compatible de celui-ci, comme agent actif, avec des véhicules et additifs pharmaceutiquement admissibles et le cas échéant, un ou plusieurs autres agents actifs pharmaceutiques.

9. Utilisation d'un composé de la formule I selon une ou plusieurs des revendications 1 à 5 et/ou d'un sel physiologiquement compatible de celui-ci, pour la préparation d'un médicament avec action bloquant les canaux K⁺ pour la thérapie ou la prophylaxie des maladies à médiation par les canaux K⁺.

10. Utilisation d'un composé de la formule I selon une ou plusieurs des revendications 1 à 5 et/ou d'un sel physiologiquement compatible de celui-ci, pour la préparation d'un médicament pour la thérapie ou la prophylaxie de troubles du rythme cardiaque, qui peuvent être levés par un allongement du potentiel d'action.

11. Utilisation d'un composé de la formule I selon une ou plusieurs des revendications 1 à 5 et/ou d'un sel physiologiquement compatible de celui-ci, pour la préparation d'un médicament pour la thérapie ou la prophylaxie des arythmies de rentrée.

12. Utilisation d'un composé de la formule I selon une ou plusieurs des revendications 1 à 5 et/ou d'un sel physiologiquement compatible de celui-ci, pour la préparation d'un médicament pour la thérapie ou la prophylaxie des arythmies supraventriculaires.

13. Utilisation d'un composé de la formule I selon une ou plusieurs des revendications 1 à 5 et/ou d'un sel physiologiquement compatible de celui-ci, pour la préparation d'un médicament pour la thérapie ou la prophylaxie des fibrillations auriculaires ou de troubles auriculaires.

14. Composition pharmaceutique, contenant une quantité active d'au moins un composé de la formule I selon une ou plusieurs des revendications 1 à 5 et/ou un sel physiologiquement compatible de celui-ci, ainsi qu'un bêta-bloquant, comme agents actifs, avec des véhicules et additifs pharmaceutiquement admissibles.

15. Composition pharmaceutique, contenant une quantité active d'au moins un composé de la formule I selon une ou plusieurs des revendications 1 à 5 et/ou un sel physiologiquement compatible de celui-ci, ainsi qu'un agent bloquant les canaux IKs, comme agents actifs, avec des véhicules et additifs pharmaceutiquement admissibles.
